# EUROPEAN PATENT APPLICATION

(11) **EP 4 144 755 A1**
(43) Date of publication of application: **08.03.2023**
(21) Application number: 22158992.2
(22) Date of filing: 25.02.2022
(51) Int. Cl.: C07K 14/435, G07D 7/12

(54) **LABELLING COMPOSITION, METHODS AND USES THEREOF**

(30) Priority: 06.09.2021 PT 2021117444
(71) Applicant: Universidade do Minho, 4704-553 Braga (PT); Centi - Centro De Nanotecnologia E Materiais Tecnicos Funcionais e Inteligentes, 4760-034 Vila Nova de Famalicão (PT)
(72) Inventor: DA QUINTA VIDAL AGUIAR, Tatiana Sofia, 3750-583 MACINHATA DO VOUGA (PT); SILVA ALVES OLIVEIRA, Juliana Andreia, 4760-034 VILA NOVA DE FAMALICÃO (PT); OLIVEIRA BARROS, Ana Rita, 4760-034 VILA NOVA DE FAMALICÃO (PT); DE CARVALHO ROQUE BOUÇA, Verónica, 4760-034 VILA NOVA DE FAMALICÃO (PT); LOUREIRO RAMADA, David João, 4760-034 VILA NOVA DE FAMALICÃO (PT); DA COSTA FREITAS, Ana Isabel, 4460-428 SENHORA DA HORA (PT); ALVES RIBEIRO DOMINGUES, Lucília Maria, 4715-065 BRAGA (PT)
(74) Representative: Patentree

(57) **Abstract**

The present disclosure relates to a labelled article comprising a substrate selected from a list consisting of at least one of the following substrates: paper, cork, leather, yarns, threads, textiles, fabrics, a particle, or combinations thereof; a reversibly switchable fluorescent peptide; and a binder peptide, preferably more than one binder, wherein the binder binds the fluorescent peptide to the substrate, preferably, wherein the fluorescent peptide and the binder peptide are bound and form a fusion protein. A labelling composition for marking and identifying an article, and an ink, paste, film, or gel comprising said composition are also disclosed.

## Description

### TECHNICAL FIELD

The present disclosure relates to an anti-counterfeiting technology, especially referring to anti-counterfeiting markers based on reversibly switchable fluorescent proteins with unique fluorescence properties, and methods for producing, detecting, and using such markers. Namely, a labelling composition, a labelled substrate, and methods thereof.

### BACKGROUND

Counterfeiting refers to the action of making an imitation of something valuable with the intention to deceive or defraud. Historically, it was primarily focused on money and documents, but nowadays counterfeiting is an ever-growing problem to a multitude of industries, causing financial losses and devastating consequences to many organizations and consumers.

As the rate and extent of the problem of global counterfeiting grows, it is increasingly important for manufacturers to protect their products by taking measures to prevent and deter counterfeiting. For that, many anti-counterfeiting security solutions have been developed, used and proposed. Traditionally, these can be categorized into five different platforms - overt, covert, semi-covert, forensic and digital - each representing a different/specific level of security.

Overt platforms comprise visual or tactile security features that can be identified without devices. Holograms, color-shifting inks, simple codes and watermarks (in paper) are examples of such features. Covert platforms include hidden security features that can only be detected by using specialized readers or proprietary devices. Among these are advanced infrared (IR) markers, ultraviolet (UV)-fluorescent inks, laser-activating inks, invisible markers embedded in substrates and complex codes. Semi-covert platforms comprise visible or semi-visible features that may go unnoticed until they are revealed. Examples of such features include pantographs, micro text, scrambled indicia, metameric inks, special-effect inks, coin-reactive inks and thermochromic inks. Forensic features, such as molecular markers, DNA-type inks, nanoparticle coatings and chemical or spectral signatures, are only detectable with specific laboratory equipment. Digital security features, which can be overt or covert, relate to numeric values or digital symbology that enable the verification of the authenticity of the product.

Of the anti-counterfeiting solutions listed above, the prevalent technology implemented, and most often recommended, consists of hidden image technology based on covert features virtually impossible to duplicate, such as those of specialized inks and/or substrate markers. These markers are often supplied in the form of standard press-ready inks (including over-print varnishes and coatings) suitable for direct application or as concentrates suitable for blending into substrates. In this way, packaging or labels can be printed with these security inks or coatings without having to materially adjust or modify any existing printing equipment or existing processes.

Specialized detection devices are specifically tuned to look for unique optical or other physical properties of a marker before internal algorithms make a "yes/no" determination. The most widely available optical markers are based on fluorescent materials/molecules detectable only by dedicated readers.

Fluorescence is the emission of light by matter after it is irradiated with light of a specific wavelength. The emitted light is generally of longer wavelength than that of the source light. Fluorescent inks used for security purposes are not visible to the naked eye under ambient light, their emission can only be seen or detected upon excitation with light of specific wavelength. This effect is immediate and reversible and ends once the excitation light source is turned off.

Standard type UV markers (seen with black light sources) are generally regarded as low-level security, since they can be readily detected and replicated. IR fluorescent markers are most widely used due to their high level of security. However, since such inks are easily sourced on the open market, they are also available to fraudsters. Last but not least, existing optical markers are commonly based on non-bio and/or non-eco-friendly molecules or materials, which is a relevant drawback with the current growing environmental concerns.

Therefore, there is the need for innovative high-level security solutions based on sustainable fluorescent markers with a unique ID and virtually impossible to imitate.

These facts are disclosed in order to illustrate the technical problem addressed by the present disclosure.

### GENERAL DESCRIPTION

The present disclosure relates to an anti-counterfeiting marker based on fluorescent proteins that switch between two or more stable states when irradiated with light of appropriate wavelengths, wherein the switching between states is fast and can be reversible. The fluorescent protein moiety of such markers can be fused to a surface binding protein moiety, to allow direct binding/immobilization onto/into specific matrices, and/or encapsulated into one or more protective layer(s). Such markers have unique fluorescent properties and are completely biocompatible and eco-friendly. A device to detect such markers is also described.

An aspect of the present disclosure relates to a labelling composition and labelled articles, wherein said labelling composition is able to mark a substrate (organic or inorganic), maintaining the fluorescence properties during a long period of time. When applied to a substrate, the labelling composition of the present disclosure is surprisingly resistant to mechanical, thermal and chemical abrasion, namely to washing, and endures exposure to direct sun light for some time. Surprisingly, the composition of the present disclosure can be used as a switchable marker.

An aspect of the present disclosure relates to a labelled article comprising:
a substrate selected from a list consisting of at least one of the following substrates: paper, cork, leather, yarns, threads, textiles, fabrics, a particle, or combinations thereof; a fluorescent peptide wherein the peptide sequence of the fluorescent peptide comprises at least a sequence 90% identical to the sequences of the following list: SEQ ID No. 1, SEQ ID No. 2, SEQ ID No. 3, SEQ ID No. 4, SEQ ID No. 5, SEQ ID No. 6; and
a binder peptide wherein the peptide sequence of the binder comprises at least a sequence 90% identical to the sequences of the following list SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, or mixtures thereof; preferably more than one binder; wherein the binder binds the fluorescent peptide to the substrate,
preferably, wherein the fluorescent peptide and the binder peptide are bound and form a fusion protein.

In an embodiment, the fluorescent peptide comprises at least a sequence 95% identical to the sequences of the following list: SEQ ID No. 1, SEQ ID No. 2, SEQ ID No. 3, SEQ ID No. 4, SEQ ID No. 5, SEQ ID No. 6. Preferably 96%, 97%, 98%, 99% or 100% identical to the sequences of the following list: SEQ ID No. 1, SEQ ID No. 2, SEQ ID No. 3, SEQ ID No. 4, SEQ ID No. 5, SEQ ID No. 6.

In an embodiment, the binder peptide comprises at least a sequence 95% identical to the sequences of the following list: SEQ ID No. 7, SEQ ID No. 8, SEQ ID No. 9, SEQ ID No. 10, SEQ ID No. 11, SEQ ID No. 12, SEQ ID No. 13, SEQ ID No14, SEQ ID No. 15, or mixtures thereof. Preferably 96%, 97%, 98%, 99% or 100% identical to the sequences of the following list: SEQ ID No. 7, SEQ ID No. 8, SEQ ID No. 9, SEQ ID No. 10, SEQ ID No. 11, SEQ ID No. 12, SEQ ID No. 13, SEQ ID No14, SEQ ID No. 15, or mixtures thereof.

In an embodiment, the fusion protein comprises at least a sequence 90% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof.

In an embodiment, the fusion protein comprises at least a sequence 95% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof. Preferably 96%, 97%, 98%, 99% or 100% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof.

Methods for the alignment of sequences for comparison are well known in the art, such methods include GAP, BESTFIT, BLAST, FASTA and TFASTA. GAP uses the algorithm of Needleman and Wunsch¹ to find the global (over the whole the sequence) alignment of two sequences that maximizes the number of matches and minimizes the number of gaps. The BLAST algorithm² calculates percent sequence identity and performs a statistical analysis of the similarity between the two sequences. The software for performing BLAST analysis is publicly available through the National Centre for Biotechnology Information (NCBI). Global percentages of similarity and identity may also be determined using one of the methods available in the MatGAT software package³. Minor manual editing may be performed to optimize alignment between conserved motifs, as would be apparent to a person skilled in the art. The sequence identity values, which are indicated in the present subject matter as a percentage were determined over the entire amino acid sequence, using BLAST with the default parameters.

In an embodiment, the substrate is selected from a list comprising yarns, threads, textiles, and fabrics.

In an embodiment, the substrate is a fabric made of natural cellulosic fibers, or natural animal fibers, or synthetic fibers, or man-made cellulosic fibers, or mixtures thereof.

In an embodiment, the article is a clothing article, a footwear article, a headwear article, a watch article, a bill, a book, or an identity document.

In an embodiment, the fusion protein to textile ratio ranges from 0.1 to 1 mg/g, preferably 0.2 to 0.5 mg/g, i.e., the ratio between the fluorescent peptide or fused protein (in mg) and the mass of textile (in g).

In an embodiment, the fusion protein comprises at least a sequence 90% identical to SEQ ID No. 19, SEQ ID No. 27, SEQ ID No. 30, SEQ ID No. 38, SEQ ID No. 44, or SEQ ID No. 50; preferably 95%, 96%, 97%, 98%, 99% or 100% identical.

In an embodiment, the article may further comprise a particle, preferably a microparticle or nanoparticle.

In an embodiment, the nanoparticle is a silicon dioxide nanoparticle, a mesoporous silicon dioxide nanoparticle, or a chitosan nanoparticle.

In an embodiment, the fusion protein comprises at least a sequence 90% identical to SEQ ID No. 17, SEQ ID No. 18, SEQ ID No. 26, SEQ ID No.29, SEQ ID No.37, SEQ ID No. 43, or SEQ ID No. 49; preferably 95%, 96%, 97%, 98%, 99% or 100% identical, wherein the peptide is encapsulated, or immobilized on the silicon dioxide nanoparticle, preferably on mesoporous silicon dioxide nanoparticle.

Another aspect of the present disclosure relates to the use of a peptide sequence as a reversibly switchable fluorescent marker in clothing, footwear, headwear, watches, bills, identity documents, books, wherein the peptide sequence comprises at least a sequence 90% identical to the sequences of the following list: SEQ ID No. 1, SEQ ID No. 2, SEQ ID No. 3, SEQ ID No. 4, SEQ ID No. 5, SEQ ID No. 6, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof. Preferably 95%, 96%, 97%, 98%, 99% or 100% identical. More preferably, the peptide sequence comprises at least a sequence 90%, 95%, 96%, 97%, 98%, 99% or 100% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof.

Another aspect of the present disclosure relates to a labelling composition for marking and identifying an article comprising:
a fluorescent peptide, wherein the peptide sequence comprises at least a sequence 90% identical to the sequences of the following list: SEQ ID No. 1, SEQ ID No. 2, SEQ ID No. 3, SEQ ID No. 4, SEQ ID No. 5, SEQ ID No. 6; preferably 95% identical or 100% identical;
a binder peptide wherein the peptide sequence comprises at least a sequence 90% identical to the sequences of the following list SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, or mixtures thereof; preferably 95% identical or 100% identical; and
wherein the binder peptide and the fluorescent peptide are fused.

In an embodiment, the fusion protein comprises at least a sequence 90% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof; preferably 95% identical or 100% identical.

In an embodiment, the concentration of the fusion protein ranges between 100 to 1000 mg/L, preferably 300 to 700 mg/L.

In an embodiment, the labelling composition may further comprise a particle, or a gel.

In an embodiment, the fluorescent fusion protein is encapsulated inside the particle, or immobilized on the surface of the particle.

In an embodiment, the particle is a mesoporous silicon dioxide nanoparticle, a mesoporous silicon dioxide nanoparticle with a silica shell, an amorphous silicon dioxide nanoparticle, an amorphous silicon dioxide microparticle, a chitosan nanoparticle, a chitosan microparticle, a chitosan nanoparticle with silica shell, a chitosan microparticle with silica shell, an iron (II, III) oxide nanoparticle, an iron (II, III) oxide nanoparticle with silica shell, an iron (II, III) oxide nanoparticle with chitosan shell. Preferably, a mesoporous silicon dioxide nanoparticle, a mesoporous silicon dioxide nanoparticle with a silica shell, an amorphous silicon dioxide nanoparticle or a chitosan nanoparticle.

In an embodiment, the gel is a chitosan gel, or a trimethylolpropane ethoxylate:poly(methyl methacrylate) blend.

Another aspect of the present disclosure relates to an ink, paste, film, or gel comprising the composition described in the present disclosure.

Another aspect of the present disclosure relates to a method for obtaining a textile article marked with the labelling composition described in the present disclosure, comprising the following steps:
saturating the article with a labelling composition for marking and identifying an article, comprising a fluorescent peptide, a binder peptide and a suitable solvent;
squeezing the saturated article;
drying the squeezed article at a temperature ranging from 20 to 25 °C for 24h, or at 80 °C for 4 minutes;
wherein the fluorescent peptide sequence comprises at least a sequence 90% identical to the sequences of the following list: SEQ ID No. 1, SEQ ID No. 2, SEQ ID No. 3, SEQ ID No. 4, SEQ ID No. 5, SEQ ID No. 6; preferably 95% identical or 100% identical;
wherein the binder peptide sequence comprises at least a sequence 90% identical to the sequences of the following list SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, or mixtures thereof; preferably 95% identical or 100% identical; and
wherein the binder peptide and the fluorescent peptide are fused.

### BRIEF DESCRIPTION OF THE DRAWINGS

The following figures provide preferred embodiments for illustrating the disclosure and should not be seen as limiting the scope of invention.

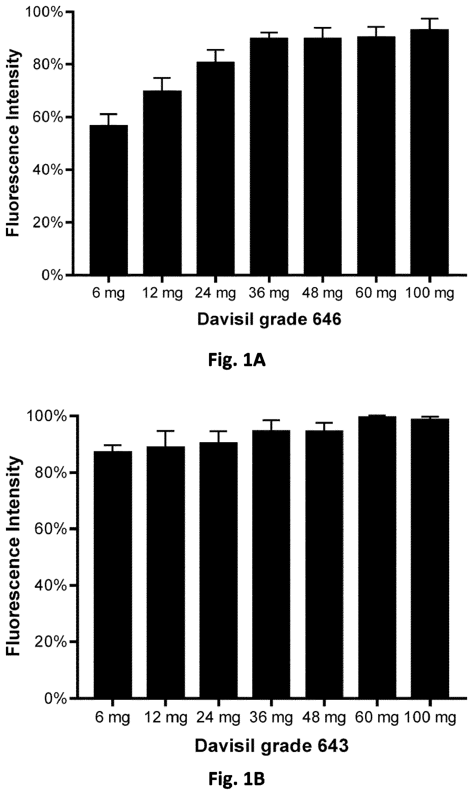

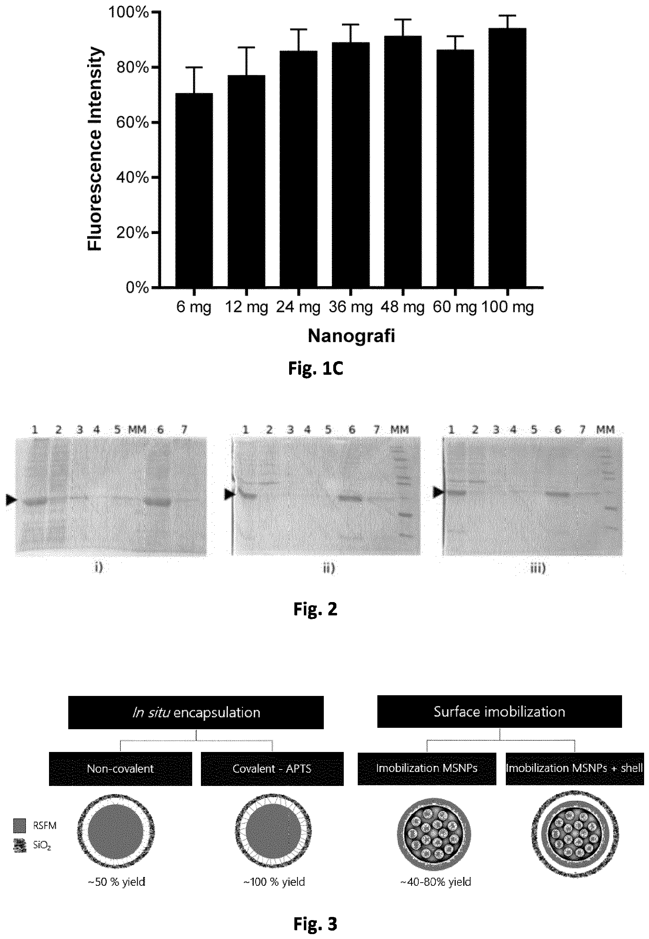

**Figure 1****:** Embodiment of the adsorptive capacity of the silica particles, determined by the amount of pure His6-rsEGFP2 (SEQ ID No. 16) adsorbed onto growing amounts of (A) mesoporous Davisil grade 646, (B) mesoporous Davisil grade 643 and (C) amorphous Nanografi silicon dioxide particles. Fluorescence intensity was normalized to an arbitrary and maximum value of 1, which stands for the fluorescence measured in the loaded solution (0.25 mg/mL) and corresponds to 100 %. Bars represent the average reading of 3 to 5 independent experiments (± SEM).
**Figure 2****:** Comparative analysis of i) His6-rsEGFP2 (SEQ ID No. 16), ii) His6-rsEGFP2-CotB1p (SEQ ID No. 17), and iii) His6-rsEGFP2-Car9 (SEQ ID No. 18) one-step purification and immobilization by SDS-PAGE. Clarified cell extracts were purified using in-house mesoporous silicon dioxide nanoparticles (NPs) as an adsorbent. Lane 1: cleared lysate; lane 2: unbound fraction; lanes 3-5: wash fractions; lanes 6-7: purified bound fractions; MM: molecular marker. The arrow indicates the target protein.
**Figure 3****:** Schematic representation of the entrapment methods used to encapsulate/immobilize the reversibly switchable fluorescent proteins (RSFPs).

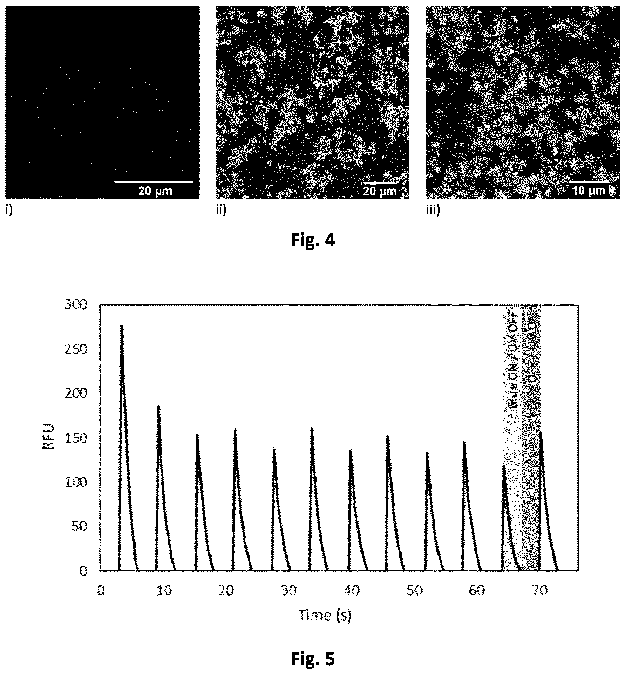

**Figure 4****:** Embodiment of fluorescence characterization by confocal fluorescence microscopy of His6-rsEGFP2 (SEQ ID No. 16) covalently encapsulated in silicon dioxide (SiO₂) nanopartticles (NPs): i) control SiO₂ NPs (no His6-rsEGFP2, scale bar - 20 µm); ii) His6-rsEGFP2-APTS@SiO₂ NPs one month after encapsulation (scale bar - 20 µm); iii) His6-rsEGFP2-APTS@SiO₂ NPs two months after encapsulation (scale bar - 10 µm).
**Figure 5****:** Embodiment of the photoswitching performance of His6-rsEGFP2 (SEQ ID No. 16) covalently encapsulated nanoparticles: fluorescence emission (RFU) over time (cycles of 3 s exposure to UV LED, 395-405 nm, followed by 3 s excitation with blue LED, 450-510 nm).

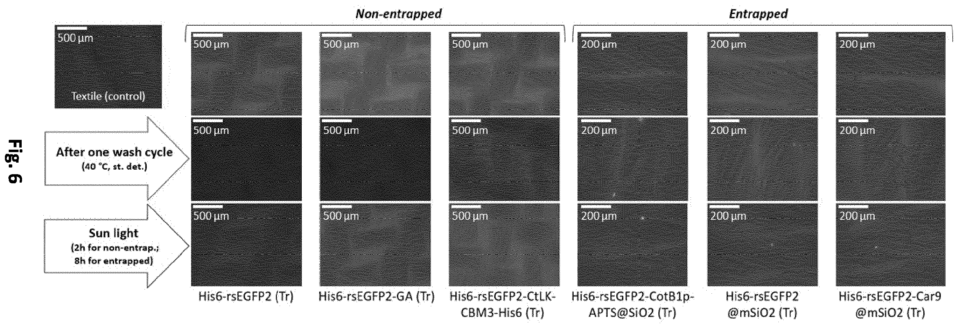

**Figure 6****:** Embodiment of fluorescence emission of textiles functionalized with non-entrapped rsEGFP2 (His6-rsEGFP2, SEQ ID No. 16; His6-rsEGFP2-GA, SEQ ID No. 16 crosslinked with glutaraldehyde; or His6-rsEGFP2-CtLK-CBM3-His6, SEQ ID No. 19) (scale bar - 500 µm), different entrapped configurations of rsEGFP2 (His6-rsEGFP2-CotB1p-APTS@SiO₂, SEQ ID No. 17; His6-rsEGFP2@mSiO₂, SEQ ID No. 16; or His6-rsEGFP2-Car9@mSiO₂, SEQ ID No. 18) (scale bar - 200 µm) and that of the corresponding textile control (scale bar - 500 µm), dried at room temperature and analyzed by optical fluorescence microscopy. First row - after functionalization; second row - after one wash cycle at 40°C with a phosphate-based reference detergent without optical brighteners (st. det. - standard detergent); third row - after 2 h of light exposure for non-entrapped and 8 h of light exposure for entrapped.

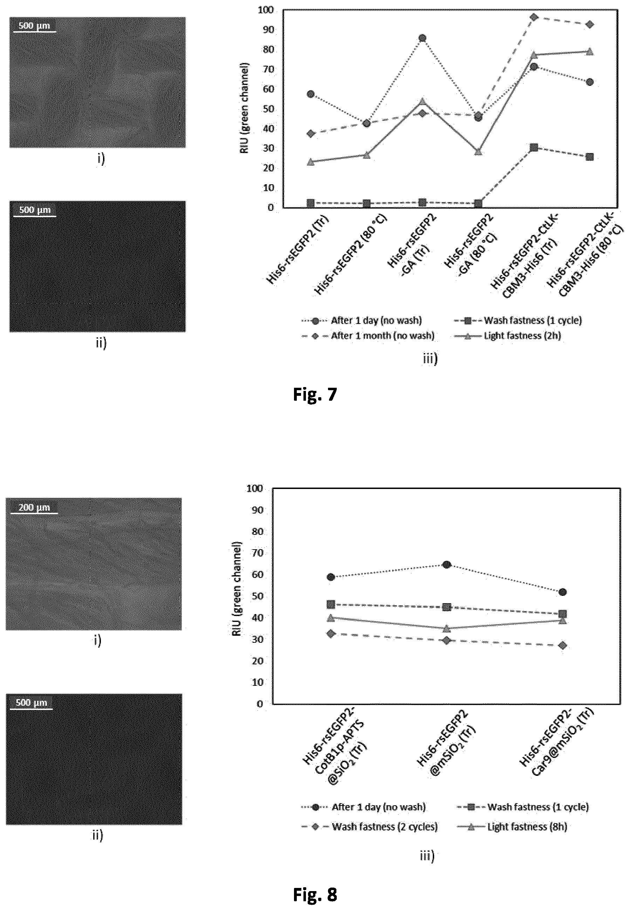

**Figure 7****:** Embodiment of the fluorescence emission of the textiles functionalized with non-entrapped rsEGFP2, dried at room temperature and at 80 °C: i) example of an optical fluorescence microscopy image of a textile sample functionalized with His6-rsEGFP2 (SEQ ID No. 16) (scale bar - 500 µm); ii) and that of the corresponding textile control (non-functionalized cotton-based textile) (scale bar - 500 µm); iii) quantification of intensity in the green scale (RIU) for textile sample functionalized with His6-rsEGFP2 (SEQ ID No. 16), His6-rsEGFP2-GA (SEQ ID No. 16 crosslinked with glutaraldehyde) or His6-rsEGFP2-CtLK-CBM3-His6 (SEQ ID No. 19) (RIU = 0 refers to the textile control).
**Figure 8****:** Embodiment of fluorescence emission of the textiles functionalized with different entrapped configurations of rsEGFP2, dried at room temperature: i) example of an optical fluorescence microscopy image of a cotton-based textile sample functionalized with His6-rsEGFP2@mSiO₂ (SEQ ID No. 16) (scale bar - 200 µm); ii) and that of the corresponding textile control (non-functionalized cotton-based textile) (scale bar - 500 µm); iii) quantification of intensity in the green scale (RIU) for textile sample functionalized with His6-rsEGFP2-CotB1p-APTS@SiO₂ (SEQ ID No. 17), His6-rsEGFP2@mSiO₂ (SEQ ID No. 16) or His6-rsEGFP2-Car9@mSiO₂ (SEQ ID No. 18) (RIU = 0 refers to the textile control).

### DETAILED DESCRIPTION

The present disclosure aims to provide anti-counterfeiting markers based on reversibly switchable fluorescent proteins (RSFPs). In addition to the standard fluorescent properties, RSFPs can be reversibly modulated by irradiation with light. Specifically, the disclosed markers can be repeatedly photoswitched between fluorescent and non-fluorescent states in a fast way by irradiation with light of two different and specific wavelengths.

In an embodiment, the wavelength of light used for exciting fluorescence in RSFPs is usually identical to one of the wavelengths used to switch between fluorescent states, but fluorescence excitation can also be decoupled from switching. Some RSFPs can also display photoconvertible properties, i.e., can irreversibly switch the fluorescence emission and/or absorption from one wavelength region to another wavelength region by irradiation with light of appropriate wavelength. In addition to their highly complex and unique features, RSFPs also display excellent biological compatibility.

Nowadays, RSFPs are commonly used as biological markers in super-resolution fluorescent microscopy, and its fluorescent properties are mainly characterized using high-cost and highly specialized equipment, limiting its widespread use. Therefore, the present disclosure also aims at providing a more practical and convenient method to inspect the unique features of these markers.

In an embodiment, the eco-sustainable anti-counterfeiting markers are produced by nanoencapsulation of RSFPs obtained by biotechnological means in biocompatible matrices, which has the double advantage of improving their thermal and photo stability and of facilitating their incorporation in different substrates.

In a further embodiment, RSFPs biologically fused to peptides that improve their binding to silica or cotton fibers were also used.

In an embodiment, non-covalent and covalent entrapment strategies were employed in the immobilization and/or encapsulation of the RSFP into biocompatible matrices and their corresponding efficiencies were compared. The fluorescent properties of the produced markers were detected by spectrofluorimetry, and their photoswitching performance was accessed with a dedicated equipment developed for such purpose.

In an embodiment, the application of these markers was achieved via conventional and advanced wet chemical methodologies.

In an embodiment, the production of the disclosed anti-counterfeiting labelling composition comprises the following steps: producing the RSFP, wherein the RSFP can be fused to a surface binding peptide moiety; optionally, functionalizing the RSFP with (3-Aminopropyl)triethoxysilane (APTS); immobilizing and/or encapsulating the RSFP in a nanoparticulate system, wherein the RSFP can be immobilized/encapsulated into one or more protective layers to obtain the anti-counterfeiting labelling composition; applying the anti-counterfeiting labelling composition on the article to be identified.

The present disclosure relates to an anti-counterfeiting and security printing fluorescent marker, comprising one or more fluorescent protein moieties that switch between two or more stable fluorescent states by being irradiated with light of appropriate wavelengths range. In an embodiment, the irradiation time ranges from 1 millisecond to 5 minutes, preferably from 1 second to 30 seconds, and the exposing light comprises a wavelength ranging from 350 nm to 600 nm.

In an embodiment, the fluorescent protein moiety comprises at least one of the following properties:
- switch between a fluorescent state and a non-fluorescent state, wherein one of the wavelengths required for reversibly switching the fluorescent fusion protein is equal to the wavelength for excitation of the fluorescence emission;
- switch between a fluorescent state and a non-fluorescent state, wherein the wavelengths required for reversibly switching the fluorescent fusion protein are different from the wavelength for excitation of the fluorescence emission;
- switch of the fluorescence emission and/or absorption of the fluorescent fusion protein from one wavelength region to another wavelength region by irradiation with light of appropriate wavelengths;
- switch between states is fast;
- is stable at room temperature and ambient light.

In an embodiment, the fluorescent protein moiety is fused to a surface binding protein moiety.

In an embodiment, the surface binding protein moiety comprises one or more of the following: carbohydrate-binding module (SEQ ID No. 10, SEQ ID No. 13), silica-binding peptide (SEQ ID No. 7, SEQ ID No. 8, SEQ ID No. 9, SEQ ID No. 15), iron oxide-binding peptide (SEQ ID No. 7, SEQ ID No. 8, SEQ ID No. 9), keratin-binding peptide (SEQ ID No. 11), silk-binding peptide (SEQ ID No. 12), plastic-binding peptide (SEQ ID No. 13, SEQ ID No. 14, SEQ ID No 15), or gold-binding peptide (SEQ ID NO. 14).

In an embodiment, the fluorescent fusion protein is encapsulated into one or more protective layer(s) of an immobilizing/encapsulating material to produce nano/microcapsules. In a further embodiment, the materials for immobilizing/encapsulating the fluorescent protein moiety or fusion protein can be selected from the following list: silicon dioxide, iron oxide, chitosan, alginate, polypropylene (PP), polystyrene, poly(ethylene terephthalate) (PET), poly(lactic acid) (PLA), gold, trimethylolpropane ethoxylate:poly(methyl methacrylate) polymer blend.

In an embodiment, the anti-counterfeiting markers or anti-counterfeiting labelling composition can be applied on paper, cork, leather, yarns, threads, textiles, or fabrics, preferably yarns, threads, textiles, or fabrics made of natural cellulosic fibers, natural animal fibers, synthetic fibers, man-made cellulosic fibers, or mixtures thereof.

In an embodiment, the natural cellulosic fibers are selected from a list comprising cotton, flax, kapok, ramie, hemp, jute, kenaf, sisal, and phormium tenax fibres.

In an embodiment, the natural animal fibers are selected from a list comprising silk, and wool.

In an embodiment, the synthetic fibers are selected from a list comprising polypropylene (PP), poly(ethylene terephthalate) (PET), polystyrene, poly(lactic acid) (PLA), polyester, nylon, and acrylic.

In an embodiment, the man-made cellulose fibers are selected from a list comprising regenerated cellulosic fibers (rayon fibers), such as viscose, acetate, cuprammonium, lyocell and modal.

The present disclosure also relates to a system for detecting and visualizing the anti-counterfeiting and security printing fluorescent markers or anti-counterfeiting composition, comprising a source for emitting a first and a second wavelength allowing the reversible switch of the fluorescent fusion protein as well as a source for exciting the fluorescent fusion protein, and means to allow the detection and visualization of the fluorescence emitted by the excited polypeptide or fusion protein.

An aspect of the present disclosure relates to a method for detecting and visualizing the anti-counterfeiting and security printing fluorescent markers or anti-counterfeiting composition, comprising the exposition of the labeling composition to light of appropriate wavelengths for 1 millisecond to 5 minutes, and the detection/observation of a decay in the fluorescence emitted by the fluorescent fusion protein, wherein the exposing light comprises a wavelength ranging from 350 nm to 600 nm.

### - Example:

### RSFPs cloning

Gene cloning can be achieved by distinct methods known to a person skilled in the art. For example, but not limiting the scope of the invention, the methods which can be used are cloning using restriction enzymes, seamless cloning, polymerase chain reaction (PCR) cloning or recombinational cloning. In an embodiment, the genetic constructs used to recombinantly express reversibly switchable fluorescent proteins (RSFPs), preferably SEQ ID No. 1 (rsEGFP2⁴), SEQ ID No. 2 (Padron⁵), SEQ ID No. 3 (NijiFP⁶), SEQ ID No. 4 (Dreiklang⁷), SEQ ID No. 5 (rsTagRFP⁸) and SEQ ID No. 6 (mTFP0.7⁹), are generated as follows, using restriction enzyme-based cloning method.

In an embodiment, the entire coding sequence for each RSFP is custom synthesized with codons optimized for recombinant expression in *Escherichia coli,* flanked by two different restriction enzymes' recognition sites at the 5'- and 3'-end. The synthetic genes are excised from the carrying plasmids by double digestion with those enzymes and cloned into an expression vector (e.g., from the pET series) in fusion with the SEQ ID No. 7 (His6-tag) at the N-terminal, generating the plasmids expressing His6-RSFP. Using these plasmids as template, and appropriate primers, the coding sequence for the silica binding peptides SEQ ID No. 8 (CotB1p¹⁰) or SEQ ID No. 9 (Car9¹¹), flanked by two different restriction enzymes' recognition sites, is fused to the C-terminal of the His6-RSFPs by PCR. The PCR products are double digested and cloned into the expression vector (e.g., from the pET series), yielding the plasmids expressing His6-RSFP-CotB1p (SEQ ID No. 17) and His6-RSFP-Car9 (SEQ ID No. 18, SEQ ID No. 26, SEQ ID No. SEQ ID No. 29, SEQ ID No. 37, SEQ ID No. 43, or SEQ ID No. 49), respectively.

In a further embodiment, the coding sequence for the silk binding peptide SEQ ID No. 12 (pepYN42¹²) or the plastic-binding peptide SEQ ID No. 15 (LCI(KR-2)^{13,14}), flanked by two different restriction enzymes' recognition sites, is also fused to the C-terminal of the His6-RSFPs by PCR. The PCR products are double digested and cloned into the expression vector (e.g., from the pET series), yielding the plasmids expressing His6-RSFP-pepYN42 (SEQ ID No. 21, SEQ ID No. 32, SEQ ID No. 40, or SEQ ID No. 46) and His6-RSFP-LCI(KR-2) (SEQ ID No. 24, or SEQ ID No. 35), respectively.

From these plasmids, the entire coding sequence for the His6-RSFPs is excised by double digestion and cloned into the expression vector pET21a(+)_LK-CBM3¹⁵, in fusion with the cellulose/cotton/chitosan binding peptide CtLK-CBM3 from the *Clostridium thermocellum* CipA at its C-terminal, yielding the plasmids pET21a(+)_His6-RSFP-CtLK-CBM3-His6.

In an embodiment, using the plasmids expressing His6-RSFP as template and appropriate primers, the coding sequence for the SEQ ID No. 11 (keratin binding peptide KP¹⁶), the SEQ ID No. 13 (chitin/cellulose/PP binding peptide BaCBM2¹⁷) or the SEQ ID No. 14 (plastic-binding peptide TA2(M1)^{14,18}), flanked by two different restriction enzymes' recognition sites, is fused to the N-terminal of the RSFPs by PCR. The PCR products are double digested and cloned into the expression vector (e.g., from the pET series) in fusion with the RSFPs containing the SEQ ID No. 7 (His6-tag) at the C-terminal, yielding the plasmids expressing KP-RSFP-His6 (SEQ ID No. 20, SEQ ID No. 31, SEQ ID No. 39, SEQ ID No. 45), *Ba*CBM2-RSFP-His6 (SEQ ID No. 22, SEQ ID No. 33, SEQ ID No. 41, SEQ ID No. 47) and TA2(M1)-RSFP-His6 (SEQ ID No. 23, SEQ ID No. 34).

In an embodiment, all constructs are transformed and propagated in chemically competent NZY5α *E. coli* cells (NZYTech) according to manufacturer's instructions and confirmed by Sanger sequencing. For protein expression, the constructs are transformed into the *E. coli* expression strain NZYBL21(DE3) (NZYTech) according to the manufacturer instructions.

### RSFPs production and purification

In an embodiment, for the production of RSFPs, pre-cultures of *E. coli* NZYBL21(DE3) cells harboring the recombinant plasmids are grown overnight at 37 °C in 10 mL LB medium containing 50 µg/mL kanamycin or 100 µg/mL ampicillin, depending on the expression plasmid being used. These pre-cultures are then used to inoculate 500 to 1000 mL fresh LB medium supplemented with appropriate antibiotic and cells are further grown to mid exponential phase (OD600nm ^{~}0.5) before induction of recombinant protein expression with 0.2 mM isopropyl β-d-1-thiogalactopyranoside (IPTG) for 16 h at 18 °C and 150 rpm orbital shaking. After production, cells are recovered by centrifugation and lysed with NZY Bacterial Cell Lysis Buffer (NZYTech) supplemented with DNAse I (100 µg/mL), lysozyme (4 µg/mL) and phenylmethylsulfonyl fluoride (PMSF; 0.01 mM) according to the manufacturer's instructions. Soluble cell-free extracts are then collected by centrifugation and filtered through 0.22 µm hydrophilic polytetrafluoroethylene (PTFE) filters.

For purification of His6-tagged RSFPS by immobilized metal ion affinity chromatography (IMAC), the filtered cell-free extracts are loaded into 5 mL HisTrap HP prepacked columns (GE Healthcare), and purification is conducted according to the manufacturer's instructions, using 100 mM Tris-HCI and 150 mM NaCl (pH 7.5) with 60 mM imidazole as equilibration and washing buffer or with 300 mM imidazole as elution buffer.

Alternatively, one-step purification and immobilization on particles is carried out for His6-, CotBlp- or Car9-tagged RSFPs. In a typical procedure, the filtered cell-free extracts are diluted 1:5 in NZY Bacterial Cell Lysis Buffer (NZYTech) and mixed with silicon dioxide (SiO₂) nano/microparticles or iron(II,III) oxide (10) nanoparticles (NPs) at a ratio of 1 mL diluted extract to 10 mg NPs or 100 mg microparticles. After mixing for 30 minutes at room temperature, the supernatant is removed by centrifugation and the particles washed 3 times with 1 volume of 100 mM Tris-HCI and 150 mM NaCl (pH 7.5).

The purification/immobilization efficiency is analysed by SDS-PAGE using 15 % (w/v) acrylamide gels, followed by BlueSafe staining (NZYTech). When necessary, imidazole removal from proteins is performed using PD10 columns (GE Healthcare), following the manufacturer instructions. The concentration of the purified RSFPs is estimated from the absorbance at 280 nm using the corresponding theoretical molar extinction coefficients. Purified RSFPs in solution, lyophilized or immobilized onto SiO₂/IO nano/microparticles are maintained in 100 mM Tris-HCI buffer (pH 7.5) at 4 °C until their subsequent use.

In an embodiment, SiO₂ particles, commercially available or produced in-house, with distinct characteristics were used to evaluate the effect of particle characteristics on adsorption. Davisil grade 643 and 646 microparticles were specifically selected due to large pore size (15 nm) and differences in terms of size, and Nanografi selected due to their nano size (table 1).

**Table1. Characteristics of the SiO₂ particles used in this study.**

| **Silica particles** | **Davisil grade 646** | **Davisil grade 643** | **Nanografi** | **In-house particles** |
|---|---|---|---|---|
| Particle size | 250-500 µm | 35-70 µm | 55-75 nm | 42 ± 7 nm^{a} |
| Pore size | 150 Å (15 nm) | 150 Å (15 nm) | - | 36 Å (3.6 nm)^{b} |
| Surface area | 300 m²/g | 300 m²/g | 150-550 m²/g | 677-947 m²/g^{b} |
| Minimum and maximum price (EUR) per g of particle^{c} | 0.92 to 4.39 | 0.86 to 4.28 | 0.11 to 0.96 | - |

| | | | | |
|---|---|---|---|---|
| ^{a} Average and standard deviation of 13 measurements, performed by Scanning Electron Microscopy (SEM) using an NanoSEM FEI Nova 200 equipment. ^{b} Nitrogen adsorption equilibrium isotherms at -196°C determined on a Quantachrome NOVA 4200e instrument and the results were analyzed by the Brunauer-Emmett-Teller model (BET) to determine the particle's structural characteristics. ^{c} Price range determined based on the corresponding companies' website quote. | | | | |

Figure 1 depicts an embodiment of the results related to the immobilization of His6-RSFPs in different SiO₂ particles. His6-tag displays affinity towards silica, a property that had not yet been reported for this immobilization/purification tag. In an embodiment, to assess the amount of SiO₂ particles needed to fully adsorb a defined amount of His6-tagged protein, growing amounts of SiO₂ particles (from 6 mg up to 100 mg) were examined by adding ^{~}0.25 mg of pure His6-rsEGFP2 (SEQ ID No. 16) (1 mL final volume). Figure 1 depicts an embodiment of the adsorptive capacity of the SiO₂ particles and demonstrates that the percentage of His6-rsEGFP2 (SEQ ID No. 16) adsorbed onto the particles is highly dependent on the SiO₂ amount, as can be observed by the gradual increase in His6-rsEGFP2 (SEQ ID No. 16) adsorption with the increasing amount of SiO₂ particles. This trend effect was most pronounced in the Davisil grade 646 (Figure 1A)) and Nanografi (Figure 1C)) particles, which adsorbed nearly 21 % more product using a higher amount of particle (100 mg vs. 6 mg), while the binding capacity seems to be only slightly affected by growing amounts of Davisil grade 643 particles (Figure 1B)).

The maximum His-rsEGFP2 (SEQ ID No. 16) load was achieved with 6 mg of the Davisil grade 643 particles (^{~}35 µg His-rsEGFP2 (SEQ ID No. 16) per gram of silica particles), followed by the Nanografi particles (^{~}30 µg His-rsEGFP2 (SEQ ID No. 16) per gram of silica particles) and then the Davisil grade 646 particles (^{~}25 µg His-rsEGFP2 (SEQ ID No. 16) per gram of silica particles).

In an embodiment, for the His6-rsEGFP2-CotB1p (SEQ ID No. 17) and His6-rsEGFP2-Car9 (SEQ ID No. 18), slightly higher loads were achieved with 6 mg particles Davisil grade 646 particles (^{~}35 µg protein per gram of silica particles) than for His-rsEGFP2 (SEQ ID No. 16). For His-rsEGFP2 (SEQ ID No. 16), His6-rsEGFP2-CotB1p (SEQ ID No. 17) and His6-rsEGFP2-Car9 (SEQ ID No. 18) the highest loads were achieved with 6 mg of the mesoporous silica (mSiO₂) NPs produced in-house (^{~}50 µg RSFP per gram of silica particles).

In a further embodiment, one-step purification and immobilization protocols were developed using 10 mg of in-house produced mSiO₂ NPs and 1 mL of 1:5 diluted cell-free extracts of His-rsEGFP2 (SEQ ID No. 16), His6-rsEGFP2-CotB1p (SEQ ID No. 17) or His6-rsEGFP2-Car9 (SEQ ID No. 18). Figure 2 shows an embodiment of the SDS-PAGE gels of these immobilizations/purifications, where a final elution step (x2) of 15 minutes with 0.5 M arginine in 100 mM Tris-HCI buffer (pH 8.5) was used to show that besides immobilization, purification was also efficiently achieved. In these gels, it is possible to see that all tags bind specifically to the silica, allowing the separation of the tagged protein from other undesired proteins, but single tagged His6-rsEGFP2 (SEQ ID No. 16) binds less strongly to silica than doubled tagged His6-rsEGFP2-CotB1p (SEQ ID No. 17) or His6-rsEGFP2-Car9 (SEQ ID No. 18). Thus, double tagging is best for purification and immobilization onto silica in a single step, preventing protein leaching, while single His6-tagging is best for purification purposes alone, allowing better recovery of the tagged protein after elution than with double tagging.

### RSFPs entrapment

In an embodiment, the composition for anti-counterfeiting marking is produced by the entrapment of RSFPs obtained by biotechnological means in biocompatible matrices, such as silica and chitosan, which has the double advantage of improving their thermal and photo stability and of facilitating their incorporation in textiles. RSFPs biologically fused to tags that improve their binding to silica, cotton, paper, silk, wool/leather, or polystyrene/PP/PET fibers were also used.

Different means can be used to entrap the RSFPs in suitable biocompatible matrices (Figure 3). In an embodiment, the RSFPs were encapsulated *in situ,* by covalent or non-covalent methods. In another embodiment, the RSFPs were immobilized on the surface of particles, preferably mesoporous SiO₂ nanoparticles. In a further embodiment, the RSFPs were immobilized on the surface of mesoporous SiO₂ nanoparticles followed by an encapsulation in a silica shell. In a further embodiment, non-covalent and covalent entrapment strategies were optimized, and their corresponding yields compared. The entrapment protocols developed are provided bellow for an arbitrary initial RSFP amount of 10 mg.

### Non-covalent encapsulation

In an embodiment, initial entrapment tests of His6-RSFPs (fused or not to the silica affinity tags CotBlp (SEQ ID No. 8) or Car9 (SEQ ID No 9)) into SiO₂ NPs were performed using a typical one-step non-covalent encapsulation method¹⁹, where the proteins are anchored to the silica matrix through coordinate bonds between added calcium (Ca²⁺) ions and the histidine residues. For that, 10 mg of lyophilized RSFP are dissolved in 435 µL of 0.1 % CaCl₂ and a water-in-oil microemulsion is prepared by mixing the previous solution with 10.85 mL of cyclohexane, 2.56 mL of Triton X-100, 2.61 mL of n-hexanol, and by adding 145 µL of tetraethylorthosilicate (TEOS) dropwise. Then, 87 µL of 25 % ammonia is added to initiate the polymerization. After all additions, the RSFP concentration in the microemulsion is around 0.6 mg/mL. After stirring the mixture for 24 h at room temperature, 29 mL of acetone is added to breakup the microemulsion and precipitate the His6-RSFP entrapped in SiO₂ NPs (His6-RSFP@SiO₂ NPs). The reaction medium is then removed by centrifugation and the NPs are washed with 25 mM Tris-HCI buffer (pH 7.3) to solubilize the non-encapsulated protein. After 1 h, the His6-RSFP@SiO₂ NPs are recovered by centrifugation and the supernatant resulting from the centrifugation is used to quantify the non-encapsulated protein via the Bradford method. Then, the His6-RSFP@SiO₂ NPs are washed several times with ethanol and water, and stored at 4 °C in 100 mM Tris-HCI buffer (pH 7.3).

### In situ covalent encapsulation

In another embodiment, the RSFPs are covalently encapsulated in SiO₂ NPs²⁰. For this purpose, the purified His6-RSFP (fused or not to the silica affinity tags CotBlp (SEQ ID No. 8) or Car9 (SEQ ID No. 9)) is firstly functionalized with an aminosilane, such as (3-aminopropyl) trimethoxysilane (APTS) that promotes the establishment of covalent bonds between the protein core and the silica shells. In a further embodiment, for the protein functionalization with APTS, 2 mL of purified RSFP dissolved in 100 mM Tris-HCI buffer (pH 7.4) (^{~}5 mg/mL) are mixed with 26.5 mg of 1-ethyl-3-(3-dimethylaminopropyl) carbodiimide hydrochloride (EDC-HCI) and 33.5 mg of *N*-hydroxysuccinimide (NHS), and the mixture is mechanically stirred for 30 minutes. Next, 332.5 µL of APTS is added to the mixture and the reaction is vigorously stirred for 24 h at room temperature. After the reaction is finished, the functionalized protein (RSFP-APTS) is purified using a G25-column with an acidic buffer pH 6.0. The concentration of protein is quantified via the Bradford method. The APTS-functionalized RSFPs are then encapsulated via a reverse microemulsion method (water-in-oil emulsion). For this purpose, the emulsion is prepared by mixing 62.5 mL of cyclohexane, 14.75 mL of Triton X-100, 15.0 mL of *n*-hexanol, 2.50 mL of APTS-RSFP (^{~}4 mg/mL) in acidic buffer (pH 6.0), and by adding 0.84 mL of TEOS dropwise. Then, 0.54 mL of 25 % ammonia is added to the previous mixture to initiate the silica polymerization reaction. After all additions, the RSFP concentration in the microemulsion is around 0.1 mg/mL. The reaction mixture is left gently stirring at room temperature for 24 h. After the reaction is finished, 170 mL of acetone are added to break the emulsion and precipitate the RSFP-APTS@SiO₂ NPs. The mixture is left stirring for 1 h and then the reaction medium is removed by centrifugation, and the RSFP-APTS@SiO₂ NPs resuspended in 25 mM Tris-HCI buffer (pH 7.3) to solubilize the protein that was not encapsulated. After 1 h, the supernatant is recovered by centrifugation and used to quantify the non-encapsulated protein via the Bradford method. The RSFP-APTS@SiO₂ NPs are washed several times with ethanol and water, and then stored at 4 °C in 100 mM Tris-HCI buffer (pH 7.3).

For the scope and interpretation of the present disclosure the term "room temperature" relates to a temperature ranging from 15 to 30 °C, preferably 20 to 25 °C.

### Immobilization in mesoporous silica

In another embodiment, His6-RSFPs proteins are immobilized in mesoporous silica NPs, using a two-step method: (i) the immobilization of His6-RSFPs (fused or not to the silica affinity tags CotBlp (SEQ ID No. 8) or Car9 (SEQ ID No. 9)) in mesoporous silica (mSiO₂) NPs, (ii) followed by the encapsulation of the protein functionalized mSiO₂ NPs in an outer silica shell. The later step is optional, but provides further stability by entrapping the protein immobilized on the surface of the mSiO₂ NPs.

The procedure for the mSiO₂ NPs synthesis is also provided²¹, alternatively commercially available mSiO₂ NPs can be employed. In an embodiment, a solution containing 12.62 mL of water, 2.07 mL of ethanol, and 2.05 mL of a 25 % cetyltrimethylammonium chloride (CTACI) is prepared and stirred for 10 minutes. To this, 3.25 mL of triethanolamine (TEA) is added, and the mixture is further stirred until all TEA is dissolved (pH 11.3). This solution is then heated to 60 °C and 1.454 mL of TEOS is added dropwise under stirring. A white suspension develops after ten minutes, which is then let to cool to room temperature for 2 h (pH 9.3). The mesoporous material is precipitated by adding 50 mL of ethanol to the translucent, colloidal aqueous suspension and the resulting precipitate is centrifuged.

Template extraction is performed by redispersing the mesoporous silica sediment through vigorous stirring in 50 mL of an ethanolic solution of ammonium nitrate (20 g/L) and then treated for 20 minutes in an ultrasonic bath. Alternatively, the mixture can be refluxed for 1 h. This procedure is repeated until the sediment is completely transparent, preferably one or two times. To replace the ammonium ions in the structure, this operation is repeated with a solution of concentrated hydrochloric acid in ethanol (5 g/L). After template extraction, mSiO₂ NPs are washed by repeated centrifugation with pure ethanol, and then dried in an oven.

In an embodiment, for protein immobilization in mSiO₂, a stock solution (0.6 mg/mL) of His6-RSFP (fused or not to the silica affinity tags CotBlp (SEQ ID No. 8) or Car9 (SEQ ID No 9)) is prepared in 100 mM Tris-HCI buffer (pH 7.4). mSiO₂ NPs (2 mg/mL) are sonicated and dispersed in the same buffer for 30 minutes. As previously described²², 17 mL of His6-RSFP stock solution is mixed with 133 mL of mSiO₂ NPs suspension and stirred 30 minutes at 25 °C. After, protein-loaded RSFP@mSiO₂ NPs are collected by centrifugation and separated from the non-immobilized protein remaining in the supernatant, which is quantified via the Bradford method.

Optionally, but preferably, the RSFP@mSiO₂ NPs can be encapsulated into a silica shell, following a reverse microemulsion method known from the state of the art²⁰. Briefly, an emulsion is prepared by mixing 62.5 mL of cyclohexane, 14.75 mL of Triton X-100, 15.0 mL of n-hexanol, 2.50 mL of RSFP@mSiO₂ NPs (^{~}2.6 mg/mL) in acidic buffer (pH 6.0), and by adding 0.84 mL of TEOS dropwise. Then, 0.54 mL of 25 % ammonia is added to the previous mixture to initiate the silica polymerization reaction. The reaction mixture is left gently stirring at room temperature for 24 h. After the reaction is finished, 170 mL of acetone are added to break the emulsion and precipitate the RSFP@mSiO₂ NPs. The mixture is let stirring for 1 h and then the reaction medium is removed by centrifugation, and the shelled RSFP@mSiO₂ NPs are resuspended in 25 mM Tris-HCI buffer (pH 7.4) to separate the non-encapsulated protein. After 1 h, the supernatant is recovered by centrifugation and used to quantify the non-encapsulated protein via the Bradford method.

In an embodiment, to evaluate the efficiency of the entrapment methods described, the respective entrapment yields were estimated indirectly from the amount of non-encapsulated RSFP remaining in the supernatant after encapsulation/immobilization, determined using the Bradford quantification method. The results obtained for the tested RSFPs are summarized in table 2.

**Table 2. Entrapment yields (%) for the tested His6-RSFPs, with and without the silica affinity tags CotBlp (SEQ ID No. 8) or Car9 (SEQ ID No. 9), according to the different entrapment methods (number of replicas in parenthesis).**

| | Non-cov. Encap. | Cov. Encap. (APTS)^{a} | Immobilization^{a} |
|---|---|---|---|
| His6-rsEGFP2 (SEQ ID No. 16) | 13 (1) | ^{~}100 (1) | 47 ± 2 (8) |
| His6-rsEGFP2-CotB1p (SEQ ID No. 17) | 86 (1) | 99.7 ± 0.6 (3) | 33 ± 3 (4) |
| His6-rsEGFP2-Car9 (SEQ ID No. 18) | 50 (1) | ^{~}100 (1) | 84 ± 3 (8) |

| | | | |
|---|---|---|---|
| ^{a} Average and standard deviation of (n) replica. | | | |

From the results obtained for the non-covalent encapsulation of rsEGFP2 (SEQ ID No 1), is possible to conclude that the presence of the silica affinity tags (CotBlp (SEQ ID No. 8) and Car9 (SEQ ID No. 9)) effectively contributes to an increase in encapsulation yields when compared to that of the single His6-tagged protein (SEQ ID No. 16).

In what concerns the immobilization method, the His6-rsEGFP2 (SEQ ID No. 16) showed better entrapment yield via immobilization than via non-covalent encapsulation. However, there appears to be a switch in results in what concerns the RSFPs tagged with the silica binders CotBlp (SEQ ID No. 8) or Car9 (SEQ ID No. 9). It has been reported that the binding of the CotBlp (SEQ ID No. 8) tag to silica is primarily based on electrostatic interactions¹⁰ that appears to be potentiated in an encapsulation method mediated by Ca²⁺ ions, which is translated in a higher entrapment yield when compared to that of the immobilization approach. Contrarily, for the Car9 tag (SEQ ID No. 9), better yields were obtained using the immobilization approach.

In a further embodiment, the supernatant collected after the encapsulation of rsEGFP2@mSiO₂ NPs was also analyzed via the Bradford method, but no protein was detected, indicating that no protein was released from the rsEGFP2@mSiO₂ NPs during the encapsulation process. Thus, the encapsulation step was not detrimental to the entrapment yield determined for the immobilization step.

In general, the covalent encapsulation method (via functionalization with APTS) delivered the best entrapment yields. No major differences were observed between the entrapment yields of rsEGFP2 tagged with the different tags, that is, the covalent encapsulation yield was ^{~}100 % for all tags and the added effect of the affinity tag (if any) could not be detected.

### Encapsulation in chitosan

In another embodiment, chitosan (CS) oligosaccharide (MW < 5 kDa) or low molecular weight CS (MW 50-190 kDa), with deacetylation degree > 75 %, is used to encapsulate RSFPs via a coacervation/precipitation method. For protein encapsulation, a RSFP stock solution (0.6 mg/mL) is prepared in 25 mM Tris-HCI buffer (pH 8.5), and a CS stock solution (0.6 mg/mL) is prepared in 1 % HCl. In a typical procedure, the CS solution is added dropwise into 10 mL of RSFP solution and stirred at room temperature for 30 minutes. Optionally, glutaraldehyde (GA, 1 %) can be added as a covalent cross-linker and the polymerization continues for 12 h at room temperature. Finally, NPs are purified and concentrated by ultracentrifugation using a G25-column with 100 mM Tris-HCI buffer (pH 7.3). The chitosan NPs containing RSFPs (RSFPs@CS NPs) are then stored at 4 °C in 100 mM Tris-HCI buffer (pH 7.3).

### RSFP-NPs characterization

### Spectrofluorimetry

In an embodiment, the fluorescence of the different RSFP-NPs produced is measured in suspension using a spectrofluorometer (HORIBA Aqualog 800) and different excitation and emission wavelengths depending on the RSFP (see table 3).

**Table 3. Excitation (Ex) and emission (Em) wavelengths used for the spectrofluorimetric analysis of each RSFP, as well as their emitted colour and colour of the light source required for their photoswitching.**

| RSFP | Ex/Em (nm) | Emitted color | On/Off switching | |
|---|---|---|---|---|
| | | | Light Source | On/Off cycle (s) |
| rsEGFP2 (SEQ ID No. 1) | 405/535 | Green | UV/Blue | ≤10/≤10 |
| Padron (SEQ ID No. 2) | 460/540 | Green | Blue/UV | ≤30/≤30 |
| NijiFP (SEQ ID NO. 3) | 405/535 or 450/590 | Green or Orange-Red | UV/Blue or Violet-Blue/Green-Yellow | ≤30/≤30 |
| Dreiklang (SEQ ID No. 4) | 460/540 | Green-Yellow | UV/Violet | ≤30/≤30 |
| rsTagRFP (SEQ ID No. 5) | 500/610 | Red | Violet-Blue/Green-Yellow | ≤30/≤30 |
| mTFP0.7 (SEQ ID No. 6) | 405/510 | Cyan | UV/Blue | ≤300/≤300 |

### Confocal microscopy

In an embodiment, images from the different RSFP-NPs produced are acquired in a Confocal Scanning Laser Microscope (Olympus BX61, Model FluoView 1000) with an excitation laser operating at 488 nm and analyzed with the program FV10-Ver4.1.1.5 (Olympus).

### Photoswitch

In an embodiment, the photoswitch of the different RSFP-NPs produced is analyzed using an optoelectronic device developed for the purpose. The device comprises: (i) several light sources (e.g., LEDs) arranged to emit on different wavelength ranges to allow the excitation and photoswitch of the RSFPs; (ii) a light sensor arranged to detect the wavelengths emitted by the RSFPs (e.g., AMS AS7262 6-Channel Visible Spectral_ID Device with Electronic Shutter and Smart Interface); (iii) a microcontroller to control the light sources, to acquire the data from the sensor and to determine whether the photoswitching properties of the RSFPs are detectable or not; (iv) a user interface to configure the analytical parameters and to display the results; (v) an opaque casing to protect against interference from external light. The light sources are strategically positioned to avoid the direct illumination of the sensor, while allowing adequate irradiation of the sample and acquisition of its emitted fluorescent light. Optionally, optical filters can be used. The color range of the light sources used for the On/Off switching of each RSFP are indicated in table 1, as well as the duration of the On/Off switching cycle used.

In an embodiment, the spectrofluorimetric analysis of the produced RSFP-NPs confirmed that, even encapsulated/immobilized, the RSFPs maintained their fluorescence emission (under hydrated conditions). From this qualitative analysis, the His6-rsEGFP2-APTS@SiO₂ and His6-rsEGFP2-CotB1p-APTS@SiO₂, obtained by covalent encapsulation (with APTS), and the His6-rsEGFP2@mSiO₂ and His6-rsEGFP2-Car9@mSiO₂, obtained by encapsulation after immobilization in mesoporous silica, revealed better results.

In a further embodiment, the selected nanomaterials were also characterized by confocal fluorescence microscopy. Two separate analyses were performed, approximately one month and two months after encapsulation. In the period between analysis, the RSFP-NPs were kept suspended in 100 mM Tris-HCI buffer (pH 7.4) under ideal storage conditions (protected from light at 4 °C). The confocal microscopy results (example shown in figure 4) showed that the fluorescence of the materials is confined to the particles, with no leaching to the surrounding medium, confirming the effective linkage between the RSFPs and the silica matrices. Particle aggregation is also clearly noticeable in the images collected by confocal microscopy. Additionally, no significant changes were detected between the sequential analyses.

In another embodiment, to evaluate the rsEGFP2 particles' photoswitching performance, all particles obtained from RSFP entrapment using different encapsulation/immobilization approaches were subjected to consecutive cycles of 3 seconds exposure to UV LED (395-405 nm) followed by 3 seconds excitation with blue LED (^{~}450-510 nm) while recording the fluorescence emission, using a dedicated equipment developed specifically for the purpose. The fluorescence graph shown in figure 5 (example for covalently encapsulated His6-rsEGFP2-APTS@SiO₂ NPs) confirms that the protein retains its photoswitching ability when entrapped in silica. For this RSFP, SEQ ID No 1, fluorescence excitation and off photoswitch are simultaneous processes, both triggered by blue light irradiation. This is translated by the sudden increase in fluorescence emission and consequent immediate partial decay upon irradiation of the NPs with blue light. After irradiation with UV light, the fluorescence emission is recuperated. The decay was observed in all rsEGFP2 entrapped samples, but the decay was better observed in the samples obtained by the covalent approach, probably due to the fact that they retained more RSFP as a result of the better entrapment yields.

### Textile functionalization

In an embodiment, cotton-based textiles (98 % cotton, 2 % elastane) were functionalized with non-entrapped His6- and CtLK-CBM3-tagged RSFPs and with encapsulated/immobilized RSFPs that revealed the best results, namely, covalently encapsulated His6-rsEGFP2-CotB1p-APTS@SiO₂, shelled His6-rsEGFP2@mSiO₂ and shelled His6-rsEGFP2-Car9@mSiO₂ (diluted in 100 mM Tris-HCI buffer, pH 7.3, to an RSFP concentration equivalent to 600 mg/L). Functionalization was achieved at room temperature via wet chemical methodologies conventionally used in textile fabrication.

In a further embodiment, textile functionalization was performed by padding, in which the ink-saturated textile is squeezed through pad rollers at controlled pressure (3 bar) and rotation speed (2 m/min) to potentiate impregnation. When using glutaraldehyde (0.1 % wt), a typical protein crosslinker, its addition to the composition is performed immediately before the functionalization. This process is repeated twice, and the functionalized textile samples are left to dry at room temperature (Tr) for 24 h, or at 80 °C for 4 minutes.

In an embodiment, the functionalization efficiency is tested by fastness to washing at 40 °C using a domestic washing machine and phosphate-based reference detergent without optical brighteners (SDCE ECE Phosphate Reference Detergent B). The stability of the RSFP media on the textile is additionally tested by fastness to light, exposing the functionalized textile samples to direct sunlight up to 8 h. The textiles' fluorescence is checked, before and after each of these tests, by optical microscopy (OM) and using a specific fluorescence filter (excitation [450-490] nm; emission > 515 nm). The summary of the optical microscopy characterization is given on Figure 6. Appreciable fluorescence emission was detected for a RSFP to textile ratio of 0.33 mg/g.

In another embodiment, for non-entrapped RSFPs, two additional approaches were further explored to improve the link between the textile substrate and RSFP: a) the addition of an aldehyde-based crosslinker, glutaraldehyde (GA), typically used for proteins; b) and the biological fusion of the RSFP to a carbohydrate-binding module (CtLK-CBM3, (SEQ ID No. 10)) that binds to cellulose in a non-covalent way, improving its affinity to cotton fibres. In an embodiment, the fluorescence characterization of the textile samples functionalized with non-entrapped His-rsEGFP2-CtLK-CBM3-His6 (SEQ ID No. 19) and related approaches was performed using an OM. The intensity of emission in the green scale of the collected images (example on figure 7i)) was quantified with the software 'ImageJ' and graphically plotted in figure 7iii) for comparison, where the null intensity (RIU = 0) refers to the textile control (figure 7ii)).

The characterization shows the effective fluorescence emission of the textile samples when functionalized with the selected RSFP, comparatively to the textile control. Also, when crosslinked with GA, the samples show a 30 % increase in fluorescence emission, which corroborates literature reports that GA induces fluorescence when interacting with proteins. After functionalization, all samples were dried at room temperature for 24 h, or at 80 °C for 4 minutes. It was verified that higher drying temperatures have a negative effect on the samples' fluorescence emission: all samples dried at 80 °C show less fluorescence emission than the equivalent dried at room temperature (Tr). Samples functionalized with rsEGFP2 biologically fused to CBM3 (His6-rsEGFP2-CtLK-CBM3-His6 (SEQ ID No. 19)), not only present better fluorescence emission than those functionalized with His6-rsEGFP2 (SEQ ID No. 16), but also the fluorescence decrease when dried at 80 °C appears to be less significant, suggesting a greater thermal stability of these samples in comparison with those functionalized with both His6-rsEGFP2 (SEQ ID No. 16) and His6-rsEGFP2 with 0.1 % GA (His6-rsEGFP2-GA).

In another embodiment, the functionalization efficiency and the protein stability in the textile was tested by fastness to washing and fastness to light, respectively. In what concerns fastness to washing, after just one wash cycle, little or no fluorescence emission was detected on the textile samples functionalized with His6-rsEGFP2 (SEQ ID No. 16) and with His6-rsEGFP2-GA. This can be explained by the almost complete removal of the protein during the washing process due to the lack of binding between protein and textile, even when using the GA crosslinker. Additionally, this can also be associated to the fact that the textiles were washed at a higher temperature (40 °C), which was already identified as detrimental to the fluorescence emission. Surprisingly, the samples functionalized with His6-rsEGFP2-CtLK-CBM3-His6 (SEQ ID No. 19) retained about 43 % of the initial fluorescence after one wash cycle, which indicates effective linkage between fluorescent media and textile substrate.

Regarding fastness to light, His6-rsEGFP2 (SEQ ID No. 16) and His6-rsEGFP2-GA functionalized samples show a decrease in emission between 35-60 % after 2 h of direct light exposure. However, His6-rsEGFP2-CtLK-CBM3-His6 (SEQ ID No. 19) functionalized samples appear to maintain the initial emission intensity, corroborating the greater thermal stability of these samples.

In yet another embodiment, the fluorescence characterization of the functionalized samples was repeated a month after functionalization on non-washed samples that were kept at room temperature and protected from light. It was verified that His6-rsEGFP2 (SEQ ID No. 16) and His6-rsEGFP2-GA functionalized samples dried at room temperature lost a considerable amount of fluorescence emission while that of the samples dried at 80 °C remained unchanged, suggesting that the degradation that naturally occurred over one month to the non-entrapped proteins mirrored that inflicted by the thermal cure. Again, the same was not verified for the His6-rsEGFP2-CtLK-CBM-His6 (SEQ ID No. 19) functionalized samples, whose fluorescence emission seems to have even improved after one month.

In another embodiment, textile samples were also functionalized with silica nanoparticles produced by the encapsulation/immobilization of the rsEGFP2 (SEQ ID No. 1). The same methodology and functionalization conditions were applied, and functionalized textile samples were left to dry at room temperature. Particle load was estimated based on the respective entrapment yield so that the protein/textile weight ratio equated what was used for the non-entrapped proteins. The fluorescence characterization of the functionalized textile samples was equally performed using OM and the intensity of emission in the green scale of the collected images was quantified and graphically plotted as before (figure 8).

Figure 8iii) summarizes the results of three different embodiments of the present disclosure: textiles functionalized with covalently encapsulated His6-rsEGFP2-CotB1p-APTS@SiO₂ (SEQ ID No. 17), with shelled His6-rsEGFP2 immobilized in mesoporous silica (His6-rsEGFP2@mSiO₂, SEQ ID No. 16) and with shelled His-rsEGFP2-Car9 immobilized in mesoporous silica (His6-rsEGFP2-Car9@mSiO₂, SEQ ID No. 18). Results show that textile samples functionalized with entrapped RSFPs retain their fluorescent properties.

In an embodiment, the functionalization efficiency and the protein stability in the textile was tested by fastness to washing and fastness to light, respectively. In what concerns fastness to washing, 70-80 % of fluorescence emission was kept after one wash cycle, which is an improvement in relation to the 43 % previously reported for samples functionalized with non-entrapped His6-rsEGFP2-CtLK-CBM3-His6 (SEQ ID No. 19). After two wash cycles, still 45-55 % of fluorescence emission remained, which indicates that the silica matrix improved the link to the textile substrate without the addition of crosslinkers and provided added protection against the mechanical and thermal aspects of washing. Regarding fastness to light, samples were exposed to 8 h of direct sunlight, after which a decrease between 25-46 % in fluorescence emission was observed.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

### Sequence list:

### Fluorescent peptides

SEQ ID No. 1 - rsEGFP2 
MVSKGEELFTGVVPILVELDGDVNGHKFSVSGEGEGDATYGKLTLKFICTTGKLPVPWPTLVTTLAYGVLCFSRYPDHM
KQHDFFKSAMPEGYVQERTIFFKDDGNYKTRAEVKFEGDTLVNRIELKGIDFKEDGNILGHKLEYNYNSHNVYIMADKQ
KNGIKSNFKIRHNIEDGSVQLADHYQQNTPIGDGPVLLPDNHYLSTQSKLSKDPNEKRDHMVLLEFVTAAGITLGMDEL
YK
SEQ ID No. 2 - Padron 
MVSVIKPDMKIKLRMEGAVNGHPFAIEGVGLGKPFEGKQSMDLKVKEGGPLPFAYDILTMAFCYGNRVFAKYPENIVD
YFKQSFPEGYSWERSMIYEDGGICNATNDITLDGDCYIYEIRFDGVNFPANGPVMQKRTVKWELSTEKLYVRDGVLKSD
GNYALSLEGGGHYRCDFKTTYKAKKVVQLPDYHSVDHHIEIKSHDKDYSNVNLHEHAEAHSELPRQAK
SEQ ID No. 3 - NijiFP 
MVNLIKEDMRVKVHMEGNVNGHAFVIEGEGKGKPYEGTQTANLTVKEGAPLPFSYDILTTAVHYGNRVFTKYPEDIPD
YFKQSFPEGYSWERTMTFEDKGICTIRSDISLEGDCFFQNVRFKGTNFPPNGPVMQKKTLKWEPSTEKLHVRDGLLVGN
INMALLLEGGGHYLCDSKTTYKAKKVVQLPDAHFVDHRIEILGNDSDYNKVKLYEHAVARYSPLPSQAW
SEQ ID No. 4 - Dreiklang 
MVSKGEELFTGVVPILVELDGDVNGHKFSVSGEGEGDATYGKLTLKFICTTGKLPVPWPTLLTTIGYGLMCFARYPDHM
KQHDFFKSAMPEGYVQERTIFFKDDGNYKTRAEVKFEGDTLVNRIELKGIDFKEDGNILGHKLEYNHDSHNVYIMADKQ
KNGIKVNFKIRHNIEDGSVQLADHYQQNTPIGDGPVLLPDNHYLSYQSALSKDPNEKRDHMVLLEFVTAAGITLGMDEL
YK
SEQ ID No. 5 - rsTagRFP 
MVSKGEELIKENMHMKLYMEGTVNNHHFKCTSEGEGKPYEGTQTMRIKVVEGGPLPFAFDILATSFMYGSRTFINHT
QGIPDFWKQSFPEGFTWERVTTYEDGGVLTATQDTSLQDGCLIYNVKLRGVNFPSNGPVMQKKTLGWEAATEMLYP
ADGGLEGRGDMALKLVGGGHLICNLKTTYRSKNPAKNLKMPGVYFVDHRLERIKEADKETYVEQHEVAVARYCDLPSK
LGHKLN
SEQ ID No. 6 - mTFP0.7

MVSKGEETTMGVIKPDMKIKLKMEGNVNGHAFVIEGEGEGKPYDGTNTINLEVKEGAPLPFSYDILTNAFAYGNRAFT
KYPDDIPNYFKQSFPEGYSWERTMTFEDKGIVKVKSDISMEEDSFIYEIHLKGENFPPNGPVMQKKTLKWEPSTEILYVR
DGVLVGDIKHKLLLEGGGHHRVDFKTIYRAKKAVKLPDYHFVDHRIEILNHDKDYNKVTVYESAVARYSTGGMDELYK

### Binders

SEQ ID No. 7 - His6
   HHHHHH
SEQ ID No. 8 - CotB1p
   SGRARAQRQSSRGR
SEQ ID No. 9 - Car9
   DSARGFKKPGKR
SEQ ID No.10 - CtLK-CBM3 
MASTPTKGATPTNTATPTKSATATPTRPSVPTNTPTNTPANTPVSGNLKVEFYNSNPSDTTNSINPQFKVTNTGSSAIDL
SKLTLRYYYTVDGQKDQTFWCDHAAIIGSNGSYNGITSNVKGTFVKMSSSTNNADTYLEISFTGGTLEPGAHVQIQGRF
AKNDWSNYTQSNDYSFKSASQFVEWDQVTAYLNGVLVWGKELE
SEQ ID No. 11 - KP
   GGVCGPSPPCITT
SEQ ID No. 12 - pepYN42
   SYTFHWHQSWSS
SEQ ID No. 13 - BaCBM2
   ATFSVTSNWGSGYNFSIVIKNSGTTPIKNWKLEFDYNGNLTQVWDSKISSKINNHYVITNAGWNGEIPPGGS
SEQ ID No. 14 - TA2(M1)
   YSSCQPQGFNCKVRPYGLPTIPCCRGLTRLSYSPGSTYGRCQRH
SEQ ID No. 15 - LCI(KR-2)
   AIKLVQSPNGNFAASFVLDGTKWIFKSKRYDSSKRYWVGIYEVWDRK

### Fused peptides

SEQ ID No. 16 - His6-rsEGFP2 
MKHHHHHHPMVSKGEELFTGVVPILVELDGDVNGHKFSVSGEGEGDATYGKLTLKFICTTGKLPVPWPTLVTTLAYGV
LCFSRYPDHMKQHDFFKSAMPEGYVQERTIFFKDDGNYKTRAEVKFEGDTLVNRIELKGIDFKEDGNILGHKLEYNYNS
HNVYIMADKQKNGIKSNFKIRHNIEDGSVQLADHYQQNTPIGDGPVLLPDNHYLSTQSKLSKDPNEKRDHMVLLEFVT
AAGITLGMDELYK
SEQ ID No. 17 - His6-rsEGFP2-CotB1p 
MKHHHHHHPMVSKGEELFTGVVPILVELDGDVNGHKFSVSGEGEGDATYGKLTLKFICTTGKLPVPWPTLVTTLAYGV
LCFSRYPDHMKQHDFFKSAMPEGYVQERTIFFKDDGNYKTRAEVKFEGDTLVNRIELKGIDFKEDGNILGHKLEYNYNS
HNVYIMADKQKNGIKSNFKIRHNIEDGSVQLADHYQQNTPIGDGPVLLPDNHYLSTQSKLSKDPNEKRDHMVLLEFVT
AAGITLGMDELYKHMSGRARAQRQSSRGR
SEQ ID No. 18 - His6-rsEGFP2-Car9 
MKHHHHHHPMVSKGEELFTGVVPILVELDGDVNGHKFSVSGEGEGDATYGKLTLKFICTTGKLPVPWPTLVTTLAYGV
LCFSRYPDHMKQHDFFKSAMPEGYVQERTIFFKDDGNYKTRAEVKFEGDTLVNRIELKGIDFKEDGNILGHKLEYNYNS
HNVYIMADKQKNGIKSNFKIRHNIEDGSVQLADHYQQNTPIGDGPVLLPDNHYLSTQSKLSKDPNEKRDHMVLLEFVT
AAGITLGMDELYKHMDSARGFKKPGKR
SEQ ID No. 19- His6-rsEGFP2-CtLK-CBM3-His6 
MKHHHHHHPMVSKGEELFTGVVPILVELDGDVNGHKFSVSGEGEGDATYGKLTLKFICTTGKLPVPWPTLVTTLAYGV
LCFSRYPDHMKQHDFFKSAMPEGYVQERTIFFKDDGNYKTRAEVKFEGDTLVNRIELKGIDFKEDGNILGHKLEYNYNS
HNVYIMADKQKNGIKSNFKIRHNIEDGSVQLADHYQQNTPIGDGPVLLPDNHYLSTQSKLSKDPNEKRDHMVLLEFVT
AAGITLGMDELYKHMASTPTKGATPTNTATPTKSATATPTRPSVPTNTPTNTPANTPVSGNLKVEFYNSNPSDTTNSIN
PQFKVTNTGSSAIDLSKLTLRYYYTVDGQKDQTFWCDHAAIIGSNGSYNGITSNVKGTFVKMSSSTNNADTYLEISFTGG
TLEPGAHVQIQGRFAKNDWSNYTQSNDYSFKSASQFVEWDQVTAYLNGVLVWGKELEHHHHHH
SEQ ID No. 20 - KP-rsEGFP2-His6 
MGGVCGPSPPCITTMVSKGEELFTGVVPILVELDGDVNGHKFSVSGEGEGDATYGKLTLKFICTTGKLPVPWPTLVTTLA
YGVLCFSRYPDHMKQHDFFKSAMPEGYVQERTIFFKDDGNYKTRAEVKFEGDTLVNRIELKGIDFKEDGNILGHKLEYN
YNSHNVYIMADKQKNGIKSNFKIRHNIEDGSVQLADHYQQNTPIGDGPVLLPDNHYLSTQSKLSKDPNEKRDHMVLLE
FVTAAGITLGMDELYKLEHHHHHH
SEQ ID No. 21 - His6-rsEGFP2-pepYN42 
MKHHHHHHPMVSKGEELFTGVVPILVELDGDVNGHKFSVSGEGEGDATYGKLTLKFICTTGKLPVPWPTLVTTLAYGV
LCFSRYPDHMKQHDFFKSAMPEGYVQERTIFFKDDGNYKTRAEVKFEGDTLVNRIELKGIDFKEDGNILGHKLEYNYNS
HNVYIMADKQKNGIKSNFKIRHNIEDGSVQLADHYQQNTPIGDGPVLLPDNHYLSTQSKLSKDPNEKRDHMVLLEFVT
AAGITLGMDELYKHMSYTFHWHQSWSS
SEQ ID No. 22 - BaCBM2-rsEGFP2-His6 
MATFSVTSNWGSGYNFSIVIKNSGTTPIKNWKLEFDYNGNLTQVWDSKISSKINNHYVITNAGWNGEIPPGGSMVSK
GEELFTGVVPILVELDGDVNGHKFSVSGEGEGDATYGKLTLKFICTTGKLPVPWPTLVTTLAYGVLCFSRYPDHMKQHD
FFKSAMPEGYVQERTIFFKDDGNYKTRAEVKFEGDTLVNRIELKGIDFKEDGNILGHKLEYNYNSHNVYIMADKQKNGIK
SNFKIRHNIEDGSVQLADHYQQNTPIGDGPVLLPDNHYLSTQSKLSKDPNEKRDHMVLLEFVTAAGITLGMDELYKLEH
HHHHH
SEQ ID No. 23 - TA2(M1)-rsEGFP2-His6 
MYSSCQPQGFNCKVRPYGLPTIPCCRGLTRLSYSPGSTYGRCQRHMVSKGEELFTGVVPILVELDGDVNGHKFSVSGEG
EGDATYGKLTLKFICTTGKLPVPWPTLVTTLAYGVLCFSRYPDHMKQHDFFKSAMPEGYVQERTIFFKDDGNYKTRAEV
KFEGDTLVNRIELKGIDFKEDGNILGHKLEYNYNSHNVYIMADKQKNGIKSNFKIRHNIEDGSVQLADHYQQNTPIGDG
PVLLPDNHYLSTQSKLSKDPNEKRDHMVLLEFVTAAGITLGMDELYKLEHHHHHH
SEQ ID No. 24 - His6-rsEGFP2-LCI(KR-2) 
MKHHHHHHPMVSKGEELFTGVVPILVELDGDVNGHKFSVSGEGEGDATYGKLTLKFICTTGKLPVPWPTLVTTLAYGV
LCFSRYPDHMKQHDFFKSAMPEGYVQERTIFFKDDGNYKTRAEVKFEGDTLVNRIELKGIDFKEDGNILGHKLEYNYNS
HNVYIMADKQKNGIKSNFKIRHNIEDGSVQLADHYQQNTPIGDGPVLLPDNHYLSTQSKLSKDPNEKRDHMVLLEFVT
AAGITLGMDELYKHMAIKLVQSPNGNFAASFVLDGTKWIFKSKRYDSSKRYWVGIYEVWDRK
SEQ ID No. 25 - His6-Padron 
MKHHHHHHPMVSVIKPDMKIKLRMEGAVNGHPFAIEGVGLGKPFEGKQSMDLKVKEGGPLPFAYDILTMAFCYGNR
VFAKYPENIVDYFKQSFPEGYSWERSMIYEDGGICNATNDITLDGDCYIYEIRFDGVNFPANGPVMQKRTVKWELSTEK
LYVRDGVLKSDGNYALSLEGGGHYRCDFKTTYKAKKVVQLPDYHSVDHHIEIKSHDKDYSNVNLHEHAEAHSELPRQAK
SEQ ID No. 26 - His6-Padron-Car9 
MKHHHHHHPMVSVIKPDMKIKLRMEGAVNGHPFAIEGVGLGKPFEGKQSMDLKVKEGGPLPFAYDILTMAFCYGNR
VFAKYPENIVDYFKQSFPEGYSWERSMIYEDGGICNATNDITLDGDCYIYEIRFDGVNFPANGPVMQKRTVKWELSTEK
LYVRDGVLKSDGNYALSLEGGGHYRCDFKTTYKAKKVVQLPDYHSVDHHIEIKSHDKDYSNVNLHEHAEAHSELPRQAK
HMDSARGFKKPGKR
SEQ ID No. 27 - His6-Padron-CtLK-CBM3-His6 
MKHHHHHHPMVSVIKPDMKIKLRMEGAVNGHPFAIEGVGLGKPFEGKQSMDLKVKEGGPLPFAYDILTMAFCYGNR
VFAKYPENIVDYFKQSFPEGYSWERSMIYEDGGICNATNDITLDGDCYIYEIRFDGVNFPANGPVMQKRTVKWELSTEK
LYVRDGVLKSDGNYALSLEGGGHYRCDFKTTYKAKKVVQLPDYHSVDHHIEIKSHDKDYSNVNLHEHAEAHSELPRQAK
HMASTPTKGATPTNTATPTKSATATPTRPSVPTNTPTNTPANTPVSGNLKVEFYNSNPSDTTNSINPQFKVTNTGSSAI
 
DLSKLTLRYYYTVDGQKDQTFWCDHAAIIGSNGSYNGITSNVKGTFVKMSSSTNNADTYLEISFTGGTLEPGAHVQIQG
RFAKNDWSNYTQSNDYSFKSASQFVEWDQVTAYLNGVLVWGKELEHHHHHH
SEQ ID No. 28 - His6-NijiFP 
MKHHHHHHPMVNLIKEDMRVKVHMEGNVNGHAFVIEGEGKGKPYEGTQTANLTVKEGAPLPFSYDILTTAVHYGNR
VFTKYPEDIPDYFKQSFPEGYSWERTMTFEDKGICTIRSDISLEGDCFFQNVRFKGTNFPPNGPVMQKKTLKWEPSTEKL
HVRDGLLVGNINMALLLEGGGHYLCDSKTTYKAKKVVQLPDAHFVDHRIEILGNDSDYNKVKLYEHAVARYSPLPSQA
W
SEQ ID No. 29 - His6-NijiFP-Car9 
MKHHHHHHPMVNLIKEDMRVKVHMEGNVNGHAFVIEGEGKGKPYEGTQTANLTVKEGAPLPFSYDILTTAVHYGNR
VFTKYPEDIPDYFKQSFPEGYSWERTMTFEDKGICTIRSDISLEGDCFFQNVRFKGTNFPPNGPVMQKKTLKWEPSTEKL
HVRDGLLVGNINMALLLEGGGHYLCDSKTTYKAKKVVQLPDAHFVDHRIEILGNDSDYNKVKLYEHAVARYSPLPSQA
WHMDSARGFKKPGKR
SEQ ID No. 30 - His6-NijiFP-CtLK-CBM3-His6 
MKHHHHHHPMVNLIKEDMRVKVHMEGNVNGHAFVIEGEGKGKPYEGTQTANLTVKEGAPLPFSYDILTTAVHYGNR
VFTKYPEDIPDYFKQSFPEGYSWERTMTFEDKGICTIRSDISLEGDCFFQNVRFKGTNFPPNGPVMQKKTLKWEPSTEKL
HVRDGLLVGNINMALLLEGGGHYLCDSKTTYKAKKVVQLPDAHFVDHRIEILGNDSDYNKVKLYEHAVARYSPLPSQA
WHMASTPTKGATPTNTATPTKSATATPTRPSVPTNTPTNTPANTPVSGNLKVEFYNSNPSDTTNSINPQFKVTNTGSS
AIDLSKLTLRYYYTVDGQKDQTFWCDHAAIIGSNGSYNGITSNVKGTFVKMSSSTNNADTYLEISFTGGTLEPGAHVQIQ
GRFAKNDWSNYTQSNDYSFKSASQFVEWDQVTAYLNGVLVWGKELEHHHHHH
SEQ ID No. 31- KP-NijiFP-His6 
MGGVCGPSPPCITTMVNLIKEDMRVKVHMEGNVNGHAFVIEGEGKGKPYEGTQTANLTVKEGAPLPFSYDILTTAVH
YGNRVFTKYPEDIPDYFKQSFPEGYSWERTMTFEDKGICTIRSDISLEGDCFFQNVRFKGTNFPPNGPVMQKKTLKWEP
STEKLHVRDGLLVGNINMALLLEGGGHYLCDSKTTYKAKKVVQLPDAHFVDHRIEILGNDSDYNKVKLYEHAVARYSPLP
SQAWLEHHHHHH
SEQ ID No. 32 - His6-NijiFP-pepYN42 
MKHHHHHHPMVNLIKEDMRVKVHMEGNVNGHAFVIEGEGKGKPYEGTQTANLTVKEGAPLPFSYDILTTAVHYGNR
VFTKYPEDIPDYFKQSFPEGYSWERTMTFEDKGICTIRSDISLEGDCFFQNVRFKGTNFPPNGPVMQKKTLKWEPSTEKL
HVRDGLLVGNINMALLLEGGGHYLCDSKTTYKAKKVVQLPDAHFVDHRIEILGNDSDYNKVKLYEHAVARYSPLPSQA
WHMSYTFHWHQSWSS
SEQ ID No. 33 - BaCBM2-NijiFP-His6 
MATFSVTSNWGSGYNFSIVIKNSGTTPIKNWKLEFDYNGNLTQVWDSKISSKINNHYVITNAGWNGEIPPGGSMVNLI
KEDMRVKVHMEGNVNGHAFVIEGEGKGKPYEGTQTANLTVKEGAPLPFSYDILTTAVHYGNRVFTKYPEDIPDYFKQS
FPEGYSWERTMTFEDKGICTIRSDISLEGDCFFQNVRFKGTNFPPNGPVMQKKTLKWEPSTEKLHVRDGLLVGNINMA
LLLEGGGHYLCDSKTTYKAKKVVQLPDAHFVDHRIEILGNDSDYNKVKLYEHAVARYSPLPSQAWLEHHHHHH
SEQ ID No. 34 - TA2(M1)-NijiFP-His6 
MYSSCQPQGFNCKVRPYGLPTIPCCRGLTRLSYSPGSTYGRCQRHMVNLIKEDMRVKVHMEGNVNGHAFVIEGEGKG
KPYEGTQTANLTVKEGAPLPFSYDILTTAVHYGNRVFTKYPEDIPDYFKQSFPEGYSWERTMTFEDKGICTIRSDISLEGD
CFFQNVRFKGTNFPPNGPVMQKKTLKWEPSTEKLHVRDGLLVGNINMALLLEGGGHYLCDSKTTYKAKKVVQLPDAH
FVDHRIEILGNDSDYNKVKLYEHAVARYSPLPSQAWLEHHHHHH
SEQ ID No. 35 - His6-NijiFP-LCI(KR-2) 
MKHHHHHHPMVNLIKEDMRVKVHMEGNVNGHAFVIEGEGKGKPYEGTQTANLTVKEGAPLPFSYDILTTAVHYGNR
VFTKYPEDIPDYFKQSFPEGYSWERTMTFEDKGICTIRSDISLEGDCFFQNVRFKGTNFPPNGPVMQKKTLKWEPSTEKL
HVRDGLLVGNINMALLLEGGGHYLCDSKTTYKAKKVVQLPDAHFVDHRIEILGNDSDYNKVKLYEHAVARYSPLPSQA
WHMAIKLVQSPNGNFAASFVLDGTKWIFKSKRYDSSKRYWVGIYEVWDRK
SEQ ID No. 36 - His6-Dreiklang 
MKHHHHHHPMVSKGEELFTGVVPILVELDGDVNGHKFSVSGEGEGDATYGKLTLKFICTTGKLPVPWPTLLTTIGYGL
MCFARYPDHMKQHDFFKSAMPEGYVQERTIFFKDDGNYKTRAEVKFEGDTLVNRIELKGIDFKEDGNILGHKLEYNHD
SHNVYIMADKQKNGIKVNFKIRHNIEDGSVQLADHYQQNTPIGDGPVLLPDNHYLSYQSALSKDPNEKRDHMVLLEFV
TAAGITLGMDELYK
SEQ ID No. 37 - His6-Dreiklang-Car9 
MKHHHHHHPMVSKGEELFTGVVPILVELDGDVNGHKFSVSGEGEGDATYGKLTLKFICTTGKLPVPWPTLLTTIGYGL
MCFARYPDHMKQHDFFKSAMPEGYVQERTIFFKDDGNYKTRAEVKFEGDTLVNRIELKGIDFKEDGNILGHKLEYNHD
SHNVYIMADKQKNGIKVNFKIRHNIEDGSVQLADHYQQNTPIGDGPVLLPDNHYLSYQSALSKDPNEKRDHMVLLEFV
TAAGITLGMDELYKHMDSARGFKKPGKR
SEQ ID No. 38 - His6-Dreiklang-CtLK-CBM3-His6 
MKHHHHHHPMVSKGEELFTGVVPILVELDGDVNGHKFSVSGEGEGDATYGKLTLKFICTTGKLPVPWPTLLTTIGYGL
MCFARYPDHMKQHDFFKSAMPEGYVQERTIFFKDDGNYKTRAEVKFEGDTLVNRIELKGIDFKEDGNILGHKLEYNHD
SHNVYIMADKQKNGIKVNFKIRHNIEDGSVQLADHYQQNTPIGDGPVLLPDNHYLSYQSALSKDPNEKRDHMVLLEFV
TAAGITLGMDELYKHMASTPTKGATPTNTATPTKSATATPTRPSVPTNTPTNTPANTPVSGNLKVEFYNSNPSDTTNSI
NPQFKVTNTGSSAIDLSKLTLRYYYTVDGQKDQTFWCDHAAIIGSNGSYNGITSNVKGTFVKMSSSTNNADTYLEISFTG
GTLEPGAHVQIQGRFAKNDWSNYTQSNDYSFKSASQFVEWDQVTAYLNGVLVWGKELEHHHHHH
SEQ ID No. 39 - KP-Dreiklang-His6 
MGGVCGPSPPCITTMVSKGEELFTGVVPILVELDGDVNGHKFSVSGEGEGDATYGKLTLKFICTTGKLPVPWPTLLTTIG
YGLMCFARYPDHMKQHDFFKSAMPEGYVQERTIFFKDDGNYKTRAEVKFEGDTLVNRIELKGIDFKEDGNILGHKLEYN
HDSHNVYIMADKQKNGIKVNFKIRHNIEDGSVQLADHYQQNTPIGDGPVLLPDNHYLSYQSALSKDPNEKRDHMVLLE
FVTAAGITLGMDELYKLEHHHHHH
SEQ ID No. 40 - His6-Dreiklang-pepYN42 
MKHHHHHHPMVSKGEELFTGVVPILVELDGDVNGHKFSVSGEGEGDATYGKLTLKFICTTGKLPVPWPTLLTTIGYGL
MCFARYPDHMKQHDFFKSAMPEGYVQERTIFFKDDGNYKTRAEVKFEGDTLVNRIELKGIDFKEDGNILGHKLEYNHD
SHNVYIMADKQKNGIKVNFKIRHNIEDGSVQLADHYQQNTPIGDGPVLLPDNHYLSYQSALSKDPNEKRDHMVLLEFV
TAAGITLGMDELYKHMSYTFHWHQSWSS
SEQ ID No. 41 - BaCBM2-Dreiklang-His6 
MATFSVTSNWGSGYNFSIVIKNSGTTPIKNWKLEFDYNGNLTQVWDSKISSKINNHYVITNAGWNGEIPPGGSMVSK
GEELFTGVVPILVELDGDVNGHKFSVSGEGEGDATYGKLTLKFICTTGKLPVPWPTLLTTIGYGLMCFARYPDHMKQHD
FFKSAMPEGYVQERTIFFKDDGNYKTRAEVKFEGDTLVNRIELKGIDFKEDGNILGHKLEYNHDSHNVYIMADKQKNGI
KVNFKIRHNIEDGSVQLADHYQQNTPIGDGPVLLPDNHYLSYQSALSKDPNEKRDHMVLLEFVTAAGITLGMDELYKLE
HHHHHH
SEQ ID No. 42 - His6-rsTagRFP 
MKHHHHHHPMVSKGEELIKENMHMKLYMEGTVNNHHFKCTSEGEGKPYEGTQTMRIKVVEGGPLPFAFDILATSFM
YGSRTFINHTQGIPDFWKQSFPEGFTWERVTTYEDGGVLTATQDTSLQDGCLIYNVKLRGVNFPSNGPVMQKKTLGW
EAATEMLYPADGGLEGRGDMALKLVGGGHLICNLKTTYRSKNPAKNLKMPGVYFVDHRLERIKEADKETYVEQHEVAV
ARYCDLPSKLGHKLN
SEQ ID No. 43 - His6-rsTagRFP-Car9 
MKHHHHHHPMVSKGEELIKENMHMKLYMEGTVNNHHFKCTSEGEGKPYEGTQTMRIKVVEGGPLPFAFDILATSFM
YGSRTFINHTQGIPDFWKQSFPEGFTWERVTTYEDGGVLTATQDTSLQDGCLIYNVKLRGVNFPSNGPVMQKKTLGW
EAATEMLYPADGGLEGRGDMALKLVGGGHLICNLKTTYRSKNPAKNLKMPGVYFVDHRLERIKEADKETYVEQHEVAV
ARYCDLPSKLGHKLNHMDSARGFKKPGKR
SEQ ID No. 44 - His6-rsTagRFP-CtLK-CBM3-His6 
MKHHHHHHPMVSKGEELIKENMHMKLYMEGTVNNHHFKCTSEGEGKPYEGTQTMRIKVVEGGPLPFAFDILATSFM
YGSRTFINHTQGIPDFWKQSFPEGFTWERVTTYEDGGVLTATQDTSLQDGCLIYNVKLRGVNFPSNGPVMQKKTLGW
EAATEMLYPADGGLEGRGDMALKLVGGGHLICNLKTTYRSKNPAKNLKMPGVYFVDHRLERIKEADKETYVEQHEVAV
ARYCDLPSKLGHKLNHMHMASTPTKGATPTNTATPTKSATATPTRPSVPTNTPTNTPANTPVSGNLKVEFYNSNPSDT
TNSINPQFKVTNTGSSAIDLSKLTLRYYYTVDGQKDQTFWCDHAAIIGSNGSYNGITSNVKGTFVKMSSSTNNADTYLEI
SFTGGTLEPGAHVQIQGRFAKNDWSNYTQSNDYSFKSASQFVEWDQVTAYLNGVLVWGKELEHHHHHH
SEQ ID No. 45 - KP-rsTagRFP-His6 
MGGVCGPSPPCITTMVSKGEELIKENMHMKLYMEGTVNNHHFKCTSEGEGKPYEGTQTMRIKVVEGGPLPFAFDILA
TSFMYGSRTFINHTQGIPDFWKQSFPEGFTWERVTTYEDGGVLTATQDTSLQDGCLIYNVKLRGVNFPSNGPVMQKK
TLGWEAATEMLYPADGGLEGRGDMALKLVGGGHLICNLKTTYRSKNPAKNLKMPGVYFVDHRLERIKEADKETYVEQ
HEVAVARYCDLPSKLGHKLNLEHHHHHH
SEQ ID No. 46 - His6-rsTagRFP-pepYN42 
MKHHHHHHPMVSKGEELIKENMHMKLYMEGTVNNHHFKCTSEGEGKPYEGTQTMRIKVVEGGPLPFAFDILATSFM
YGSRTFINHTQGIPDFWKQSFPEGFTWERVTTYEDGGVLTATQDTSLQDGCLIYNVKLRGVNFPSNGPVMQKKTLGW
EAATEMLYPADGGLEGRGDMALKLVGGGHLICNLKTTYRSKNPAKNLKMPGVYFVDHRLERIKEADKETYVEQHEVAV
ARYCDLPSKLGHKLNHMSYTFHWHQSWSS
SEQ ID No. 47 - BaCBM2-rsTagRFP-His6 
MATFSVTSNWGSGYNFSIVIKNSGTTPIKNWKLEFDYNGNLTQVWDSKISSKINNHYVITNAGWNGEIPPGGSMVSK
GEELIKENMHMKLYMEGTVNNHHFKCTSEGEGKPYEGTQTMRIKVVEGGPLPFAFDILATSFMYGSRTFINHTQGIPD
FWKQSFPEGFTWERVTTYEDGGVLTATQDTSLQDGCLIYNVKLRGVNFPSNGPVMQKKTLGWEAATEMLYPADGGL
EGRGDMALKLVGGGHLICNLKTTYRSKNPAKNLKMPGVYFVDHRLERIKEADKETYVEQHEVAVARYCDLPS
KLGHKLNLEHHHHHH
SEQ ID No. 48 - His6-mTFP0.7 
MKHHHHHHPMVSKGEETTMGVIKPDMKIKLKMEGNVNGHAFVIEGEGEGKPYDGTNTINLEVKEGAPLPFSYDILTN
AFAYGNRAFTKYPDDIPNYFKQSFPEGYSWERTMTFEDKGIVKVKSDISMEEDSFIYEIHLKGENFPPNGPVMQKKTLK
WEPSTEILYVRDGVLVGDIKHKLLLEGGGHHRVDFKTIYRAKKAVKLPDYHFVDHRIEILNHDKDYNKVTVYESAVARYST
GGMDELYK
SEQ ID No. 49 - His6-mTFP0.7-Car9 
MKHHHHHHPMVSKGEETTMGVIKPDMKIKLKMEGNVNGHAFVIEGEGEGKPYDGTNTINLEVKEGAPLPFSYDILTN
AFAYGNRAFTKYPDDIPNYFKQSFPEGYSWERTMTFEDKGIVKVKSDISMEEDSFIYEIHLKGENFPPNGPVMQKKTLK
WEPSTEILYVRDGVLVGDIKHKLLLEGGGHHRVDFKTIYRAKKAVKLPDYHFVDHRIEILNHDKDYNKVTVYESAVARYST
GGMDELYKHMDSARGFKKPGKR
SEQ ID No. 50 - His6-mTFP0.7-CtLK-CBM3-His6

MKHHHHHHPMVSKGEETTMGVIKPDMKIKLKMEGNVNGHAFVIEGEGEGKPYDGTNTINLEVKEGAPLPFSYDILTN
AFAYGNRAFTKYPDDIPNYFKQSFPEGYSWERTMTFEDKGIVKVKSDISMEEDSFIYEIHLKGENFPPNGPVMQKKTLK
WEPSTEILYVRDGVLVGDIKHKLLLEGGGHHRVDFKTIYRAKKAVKLPDYHFVDHRIEILNHDKDYNKVTVYESAVARYST
GGMDELYKHMASTPTKGATPTNTATPTKSATATPTRPSVPTNTPTNTPANTPVSGNLKVEFYNSNPSDTTNSINPQFK
VTNTGSSAIDLSKLTLRYYYTVDGQKDQTFWCDHAAIIGSNGSYNGITSNVKGTFVKMSSSTNNADTYLEISFTGGTLEP
GAHVQIQGRFAKNDWSNYTQSNDYSFKSASQFVEWDQVTAYLNGVLVWGKELEHHHHHH

### REFERENCES:

1 S. B. Needleman, C. D. Wunsch (1970). A general method applicable to the search for similarities in the amino acid sequence of two proteins, J. Mol. Biol. 48(3):443-453 (DOI: 10.1016/0022-2836(70)90057-4).
2 S. F. Altschul, W. Gish, W. Miller, E.W. Myers, D.J. Lipman (1990). Basic local alignment search tool, J. Mol. Biol. 215(3):403-10 (DOI: 10.1016/S0022-2836(05)80360-2).
3 J.J. Campanella, L. Bitincka, J. Smalley (2003). MatGAT: An application that generates similarity/identity matrices using protein or DNA sequences, BMC Bioinformatics 4:29 (DOI: 10.1186/1471-2105-4-29).
4 T. Grotjohann, I. Testa, M. Reuss, T. Brakemann, C. Eggeling, S.W. Hell, S. Jakobs (2012). rsEGFP2 enables fast RESOLFT nanoscopy of living cells, eLife 1:e00248 (DOI: 10.7554/eLife.00248).
5 M. Andresen, A.C. Stiel, J. Fölling, D. Wenzel, A. Schönle, A. Egner, C. Eggeling, S.W. Hell, S. Jakobs (2008). Photoswitchable fluorescent proteins enable monochromatic multilabel imaging and dual color fluorescence nanoscopy, Nat. Biotechnol. 26(9):1035-1040 (DOI: 10.1038/nbt.1493).
6 V. Adam, B. Moeyaert, C.C. David, H. Mizuno, M. Lelimousin, P. Dedecker, R. Ando, A. Miyawaki, J. Michiels, Y. Engelborghs, J. Hofkens (2011). Rational design of photoconvertible and biphotochromic fluorescent proteins for advanced microscopy applications, Chem. Biol. 18(10):1241-1251 (DOI: 10.1016/j.chembiol.2011.08.007).
7 T. Brakemann, A.C. Stiel, G. Weber, M. Andresen, I. Testa, T. Grotjohann, M. Leutenegger, U. Plessmann, H. Urlaub, C. Eggeling, M.C. Wahl, S.W. Hell, S. Jakobs (2011). A reversibly photoswitchable GFP-like protein with fluorescence excitation decoupled from switching, Nat. Biotechnol. 29:942-947 (DOI: 10.1038/nbt.1952).
8 F.V. Subach, L. Zhang, T.W.J. Gadella, N.G. Gurskaya, K.A. Lukyanov, V.V. Verkhusha (2010). Red fluorescent protein with reversibly photoswitchable absorbance for photochromic FRET, Chem. Biol. 17(7):745-755 (DOI: 10.1016/j.chembiol.2010.05.022).
9 J.N. Henderson, H.-w. Ai, R.E. Campbell, S.J. Remington (2007). Structural basis for reversible photobleaching of a green fluorescent protein homologue, PNAS 104(16):6672-6677 (DOI: 10.1073/pnas.0700059104).
10 M.A.A. Abdelhamid, K. Motomura, T. Ikeda, T. Ishida, R. Hirota, A. Kuroda (2014). Affinity purification of recombinant proteins using a novel silica-binding peptide as a fusion tag, Appl. Microbiol. Biotechnol. 98(12):5677-5684 (DOI: 10.1007/s00253-014-5754-z).
11 B.L. Coyle, F. Baneyx (2014). A cleavable silica-binding affinity tag for rapid and inexpensive protein purification, Biotechnol. Bioeng. 111(10):2019-2026 (DOI: 10.1002/bit.25257).
12 Y. Nomura, V. Sharma, A. Yamamura, Y. Yokobayashi (2011). Selection of silk-binding peptides by phage display, Biotechnol. Lett. 33(5):1069-1073 (DOI: 10.1007/s10529-011-0519-6).
13 K. Rübsam, M.D. Davari, F. Jakob, U. Schwaneberg (2018). KnowVolution of the polymer-binding peptide LCI for improved polypropylene binding, Polymers 10(4):423 (DOI: 10.3390/polym10040423).
14 S. Dedisch, A. Wiens, M.D. Davari, D. Söder, C. Rodriguez-Emmenegger, F. Jakob, U. Schwaneberg (2020). Matter-tag: A universal immobilization platform for enzymes on polymers, metals, and silicon-based materials, Biotechnol. Bioeng. 117(1):49-61 (DOI: 10.1002/bit.27181).
15 C.I.P.D. Guerreiro, C.M.G.A. Fontes, M. Gama, L. Domingues (2008). Escherichia coli expression and purification of four antimicrobial peptides fused to a family 3 carbohydrate-binding module (CBM) from Clostridium thermocellum, Protein Expr. Purif. 59(1):161-168 (DOI: 10.1016/j.pep.2008.01.018).
16 C.F. Cruz, M. Martins, J. Egipto, H. Osório, A. Ribeiro, A. Cavaco-Paulo (2017). Changing the shape of hair with keratin peptides, RSC Adv. 7:51581-51592 (DOI: 10.1039/C7RA10461H).
17 J. Weber, D. Petrovic, B. Strodel, S.H.J. Smits, S. Kolkenbrock, C. Leggewie, K.-E. Jaeger (2019). Interaction of carbohydrate-binding modules with poly(ethylene terephthalate), Appl. Microbiol. Biotechnol. 103(12):4801-4812 (DOI: 10.1007/s00253-019-09760-9).
18 K. Rübsam, L. Weber, F. Jakob, U. Schwaneberg (2018). Directed evolution of polypropylene and polystyrene binding peptides, Biotechnol. Bioeng. 115(2):321-330 (DOI: 10.1002/bit.26481).
19 A. Cao, Z. Ye, Z. Cai, E. Dong, X. Yang, G. Liu, X. Deng, Y. Wang, S.-T. Yang, H. Wang, M. Wu, Y. Liu (2010). A facile method to encapsulate proteins in silica nanoparticles: encapsulated green fluorescent protein as a robust fluorescence probe, Angew. Chem. Int. Ed. Engl. 49(17):3022-3025 (DOI: 10.1002/anie.200906883).
20 Z. Cai, Z. Ye, X. Yang, Y. Chang, H. Wang, Y. Liu, A. Cao (2011). Encapsulated enhanced green fluorescence protein in silicananoparticle for cellular imaging, Nanoscale 3:1974-1976 (DOI: 10.1039/c0nr00956c).
21 K. Möller, J. Kobler, T. Bein (2007). Colloidal suspensions of mercapto-functionalized nanosized mesoporous silica, J. Mater. Chem. 17:624-631 (DOI: 10.1039/b611931j).²² J. Tu, A.L. Boyle, H. Friedrich, P.H.H. Bomans, J. Bussmann, N.A.J.M. Sommerdijk, W. Jiskoot, A. Kros (2016). Mesoporous silica nanoparticles with large pores for the encapsulation and release of proteins, ACS Appl. Mater. Interfaces 8(47):32211-32219 (DOI: 10.1021/acsami.6b11324).

## Claims

1. A labelled article comprising:
a substrate selected from a list consisting of at least one of the following substrates: paper, cork, leather, yarns, threads, textiles, fabrics, a particle, or combinations thereof;
a fluorescent peptide wherein the peptide sequence of the fluorescent peptide comprises at least a sequence 90% identical to the sequences of the following list: SEQ ID No. 1, SEQ ID No. 2, SEQ ID No. 3, SEQ ID No. 4, SEQ ID No. 5, SEQ ID No. 6; and
a binder peptide wherein the peptide sequence of the binder comprises at least a sequence 90% identical to the sequences of the following list SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, or mixtures thereof; preferably more than one binder;
wherein the binder binds the fluorescent peptide to the substrate,
preferably, wherein the fluorescent peptide and the binder peptide are bound and form a fusion protein.

2. The article according to the previous claim wherein the fusion protein comprises at least a sequence 95% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof; preferably 100% identical.

3. The article according to any of the previous claims wherein the fusion protein to textile ratio ranges from 0.1 to 1 mg/g, preferably 0.2 to 0.5 mg/g.

4. The article according to any of the previous claims wherein the fusion protein comprises at least a sequence 95% identical to SEQ ID No. 19, SEQ ID No. 27, SEQ ID No. 30, SEQ ID No. 38, SEQ ID No. 44, or SEQ ID No. 50; preferably 100% identical.

5. The article according to any of the previous claims further comprising a particle, preferably a microparticle or nanoparticle, preferably a silicon dioxide nanoparticle, a mesoporous silicon dioxide nanoparticle, or a chitosan nanoparticle.

6. The article according to previous claim 5 wherein the fusion protein comprises at least a sequence 90% identical to SEQ ID No. 17, SEQ ID No. 18, SEQ ID No. 26, SEQ ID No.29, SEQ ID No. 37, SEQ ID No. 43, or SEQ ID No. 49; preferably 95% identical or 100% identical,
wherein the peptide is encapsulated, or immobilized on the silicon dioxide nanoparticle, preferably on mesoporous silicon dioxide nanoparticle.

7. The article according to any of the previous claims wherein the substrate is selected from a list comprising yarns, threads, textiles, and fabrics; preferably a fabric made of natural cellulosic fibres, or natural animal fibres, or synthetic fibres, or man-made cellulosic fibres, or mixtures thereof.

8. The article according to any of the previous claims wherein the article is a clothing article, a footwear article, a headwear article, a watch article, a bill, a book or an identity document.

9. Use of a peptide sequence as a reversibly switchable fluorescent marker in clothing, footwear, headwear, watches, bills, identity documents, books, wherein the peptide sequence comprises at least a sequence 95% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof.

10. A labelling composition for marking and identifying an article comprising:
a fluorescent peptide, wherein the peptide sequence comprises at least a sequence 95% identical to the sequences of the following list: SEQ ID No. 1, SEQ ID No. 2, SEQ ID No. 3, SEQ ID No. 4, SEQ ID No. 5, SEQ ID No. 6; preferably 100% identical;
a binder peptide wherein the peptide sequence comprises at least a sequence 95% identical to the sequences of the following list SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, or mixtures thereof; preferably 100% identical; and
wherein the binder peptide and the fluorescent peptide are fused.

11. The labelling composition according to the previous claim wherein the fusion protein comprises at least a sequence 95% identical to the sequences of the following list: SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, or mixtures thereof; preferably 100% identical.

12. The labelling composition according to the previous claim wherein the concentration of the fusion protein ranges between 100 to 1000 mg/L, preferably 300 to 700 mg/L.

13. The labelling composition according to previous claims 10-12 further comprising a particle, or a gel; preferably wherein the fusion protein is encapsulated inside the particle or immobilized on the surface of the particle.

14. The labelling composition according to previous claims 10-13, wherein the particle is a mesoporous silicon dioxide nanoparticle, a mesoporous silicon dioxide nanoparticle with a silica shell, an amorphous silicon dioxide nanoparticle, an amorphous silicon dioxide microparticle, a chitosan nanoparticle, a chitosan microparticle, a chitosan nanoparticle with silica shell, a chitosan microparticle with silica shell, an iron (II, III) oxide nanoparticle, an iron (II, III) oxide nanoparticle with silica shell, an iron (II, III) oxide nanoparticle with chitosan shell, preferably is a mesoporous silicon dioxide nanoparticle, a mesoporous silicon dioxide nanoparticle with a silica shell, an amorphous silicon dioxide nanoparticle or a chitosan nanoparticle; or wherein the the gel is a chitosan gel or a trimethylolpropane ethoxylate:poly(methyl methacrylate) blend.

15. An ink, paste, film, or gel comprising the labelling composition described in claims 10-14.
